(19) Europäisches Patentamt
European Patent Office
Office européen des brevets

(11) **EP 4 368 609 A1**

(12) **EUROPEAN PATENT APPLICATION**

(43) Date of publication:
**15.05.2024 Bulletin 2024/20**

(21) Application number: **23207899.8**

(22) Date of filing: **06.11.2023**

(51) International Patent Classification (IPC):
***C07C 229/08*** *(2006.01)* ***C07C 233/49*** *(2006.01)*
***A61K 9/51*** *(2006.01)* ***A61P 35/00*** *(2006.01)*

(52) Cooperative Patent Classification (CPC):
**C07C 233/49; A61K 9/5123; A61P 35/00; C07C 229/08**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.11.2022 US 202263423506 P**

(71) Applicant: **Mello Biotech Taiwan Co., Ltd.**
**Taipei City 115 (TW)**

(72) Inventors:
- **CHENG, Shun-Chu**
  **115 Taipei City (TW)**
- **CHEN, Yi-An**
  **115 Taipei City (TW)**
- **LIN, Shi-Lung**
  **Arcadia, 91007 (US)**

(74) Representative: **Lang, Christian**
**LangPatent Anwaltskanzlei IP Law Firm**
**Ingolstädter Straße 5**
**80807 München (DE)**

Remarks:
The complete document including Reference Table(s) and the Sequence Listing(s) can be downloaded from the EPO website

# (54) CATIONIC LIPIDS AND USES THEREOF

(57) Disclosed herein is directed to cationic lipids and lipid nanoparticles (LNPs) comprising the same for encapsulating and delivering active pharmaceutical ingredients (API) (e.g., nucleic acids) into cells. The cationic lipid has the structure of formula (I),

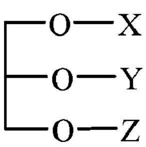 (I),

wherein, at least one of X and Y is $-(C{=}O)CH_2NH_2$, and the other X, Y, and Z are independently selected from the group consisting of $-(C{=}O)CH_2NH_2$, $-[(C{=}O)CH_2NH](C{=}O)R^1$, $-(C{=}O)R^1$, and H, wherein $R^1$ is $C_{9-25}$ alkyl, or $C_{13-21}$ alkenyl. Also encompasses herein is a method of treating a disease in a subject in need thereof, comprising administering an effective amount of the LNPs set forth above to the subject.

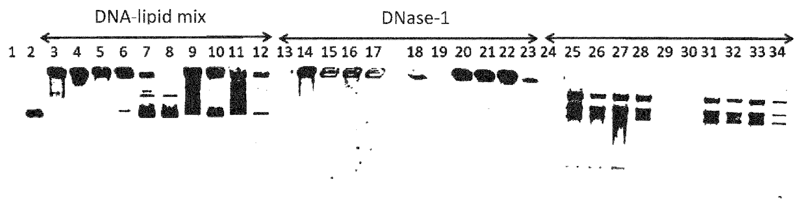


FIG. 1

EP 4 368 609 A1

**Description**

## CROSS-REFERENCES TO RELATED APPLICATIONS

This application claims priority and the benefit of U.S. Provisional Patent Application No. 63/423,506 filed November 8, 2022, the entireties of which is incorporated herein by reference.

## BACKGROUND OF THE INVENTION

### 1. FIELD OF THE INVENTION

The present disclosure relates to novel cationic lipids and their uses for enhancing the delivery of therapeutic agents. More particularly, the present disclosure relates to cationic lipids and their use for encapsulating and delivering nucleic acids into cells.

### 2. DESCRIPTION OF RELATED ART

Gene therapy is the process of introducing foreign genomic materials into host cells to elicit a therapeutic benefit. Different types of nucleic acids including DNA, plasmids-based interfering nucleic acids, small interfering nucleic acids for use in RNA interference (RNAi), antisense molecules, and aptamers, are currently being developed as therapeutic agents in gene therapy. Meanwhile, two major delivery systems - viral and non-viral systems are also being developed for encapsulating and delivering aforementioned therapeutic agents in a stable and long half-life form.

Although numerous viral and non-viral gene delivery systems are available for decades, each of them is limited in some way. For example, viral delivery systems are advantageous in their high transfection efficiency, yet they have some bio-safety concerns. Non-viral delivery systems (e.g., polymers, lipids, liposomes, micelles, dendrimers, and nanomaterials) are highly versatile in their abilities in encapsulating various sizes of nucleic acids without affecting the recipient's immune system; however, their transfection efficiency is yet to be improved.

Lipoplexes are defined as the complexes among nucleic acids and cationic lipids, and becoming one of the most studied non-viral delivery systems to date. Lipofectamine is a conventionally available cationic lipid, yet one of its components - DOSPA (2,3-dioleoyloxy-N-[2(sperminecarboxamido)ethyl]-N,N-dimethyl-1-propaniminium trifluoroacetate) was reported to have a high cytotoxicity during gene transfection.

In view of the foregoing, there exists in the related art a need of a novel, safe cationic lipid for effectively delivering therapeutic agents into cells.

## SUMMARY

The following presents a simplified summary of the disclosure in order to provide a basic understanding to the reader. This summary is not an extensive overview of the disclosure and it does not identify key/critical elements of the present invention or delineate the scope of the present invention. Its sole purpose is to present some concepts disclosed herein in a simplified form as a prelude to the more detailed description that is presented later.

The present disclosure provides novel cationic lipids and compositions thereof for encapsulating and delivering nucleic acids into cells. Also disclosed herein is a treatment of diseases via use of the present cationic lipids and/or compositions.

In one aspect, the present disclosure is directed to a cationic lipid of formula (I) shown below:

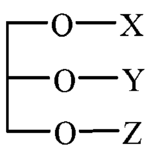

(I).

In this formula, at least one of X and Y is $-(C{=}O)CH_2NH_2$, and the other X, Y, and Z are independently selected from the group consisting of $-(C{=}O)CH_2NH_2$, $-[(C{=}O)CH_2NH](C{=}O)R^1$, $-(C{=}O)R^1$, and H, wherein $R^1$ is $C_{9-25}$ alkyl, or $C_{13-21}$ alkenyl.

According to some embodiments of the present disclosure, in the formula (I), X and Z are independently $-(C{=}O)CH_2NH_2$, Y is $-[(C{=}O)CH_2NH](C{=}O)R^1$, and $R^1$ is $C_{13-21}$ alkenyl.

According to other embodiments of the present disclosure, in the formula (I), X is - $(C{=}O)CH_2NH_2$, Y and Z are independently $-[(C{=}O)CH_2NH](C{=}O)R^1$, and $R^1$ is $C_{13-21}$ alkenyl.

According to further embodiments of the present disclosure, in the formula (I), Y is - $(C{=}O)CH_2NH_2$, X and Z are independently $-(C{=}O)R^1$, and $R^1$ is $C_{13\text{-}21}$ alkenyl.

According to still other embodiments of the present disclosure, in the formula (I), X and Z are independently $-(C{=}O)CH_2NH_2$, Y is $-(C{=}O)R^1$, and $R^1$ is $C_{13\text{-}21}$ alkenyl.

According to further embodiments of the present disclosure, in the formula (I), X is - $(C{=}O)CH_2NH_2$, Y is H, Z is $-(C{=}O)R^1$, and $R^1$ is $C_{13\text{-}21}$ alkenyl.

Preferably, a subset of the cationic lipid of formula (I) is selected from the group consisting of

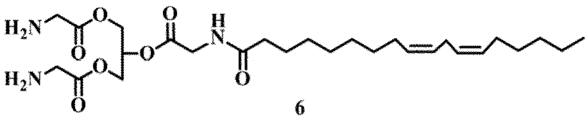

6

,

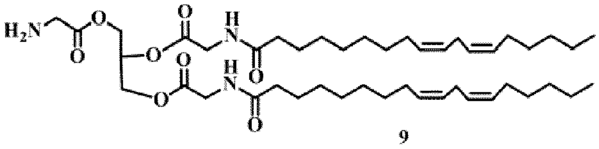

9

,

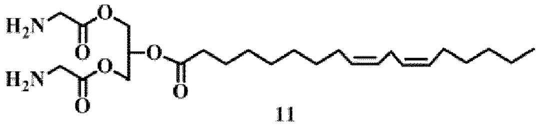

11

,

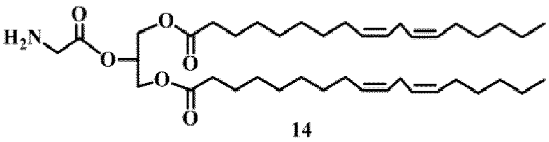

14

,

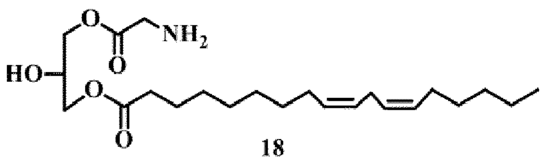

18

,

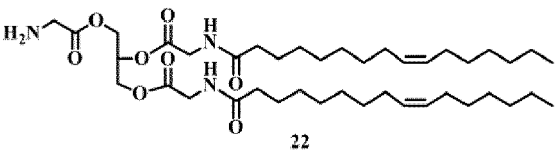

22

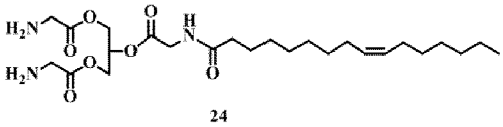

24

, and .

Another aspect of the present disclosure is directed to a lipid nanoparticle (LNP) comprising the present cationic lipid, a non-cationic lipid, and an active pharmaceutical ingredient (API).

According to some embodiments of the present disclosure, the API disposed in the present LNP may be a nucleic acid, a peptide, a polypeptide, a protein, a carbohydrate, a proteoglycan, a glycoprotein, or a combination thereof.

Example of the nucleic acid suitable for use in the present LNP includes, but is not limited to, mitochondrial DNA (mtDNA), chloroplast DNA (cpDNA), plasmid, messenger RNA (mRNA), small interfering RNA (siRNA), ribosomal RNA (rRNA), transfer RNA (tRNA), microRNA (miRNA), and aptamer. Preferably, the miRNA is a precursor miRNA or a mature miRNA.

In some preferred embodiments of the present disclosure, in the present LNP, the cationic lipid may be selected from the group consisting of

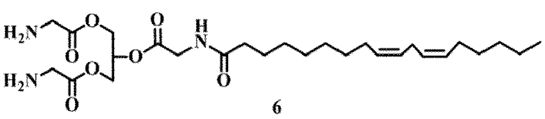

6

,

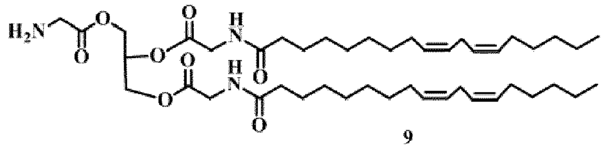

9

,

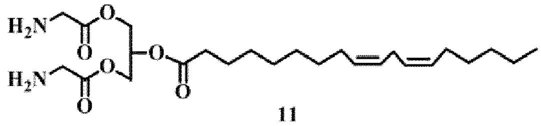

11

,

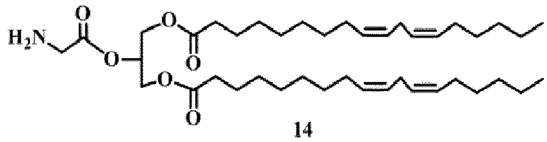

14

,

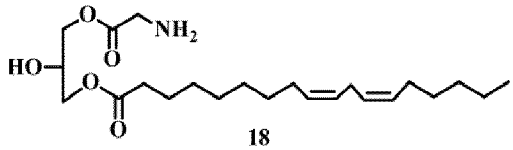

18

,

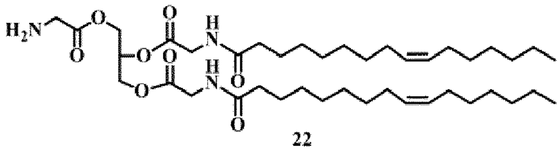

22

,

and

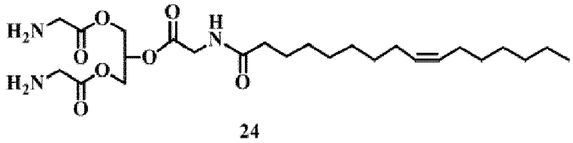

24

;

and the API is the miRNA comprising a sequence of SEQ ID NO:1. In working embodiments, the present miRNA has a sequence of SEQ ID NO:2, 3, 4, or 5

In other preferred embodiments of the present disclosure, in the LNP, the cationic lipid is

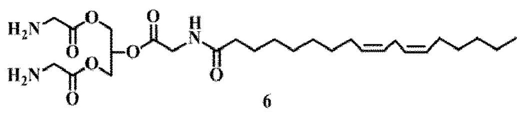

6

or

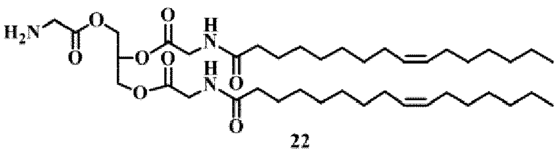

22

;

and the API is the siRNA having a sequence of SEQ ID NO: 6 or 7.

According to some embodiments of the present disclosure, the non-cationic lipids comprised in the present LNP may be a helper lipid, a phospholipid, a PEGylated lipid, a PEGylated phospholipid, or a combination thereof. Examples of the helper lipid suitable for use in the present disclosure include, but are not limited to, dioleoylphosphatidylethanolamine (DOPE), dipalmitoylphosphatidylcholine (DPPC), distearoylphosphatidylethanolamine (DSPE), dimyristoylphosphatidylethanolamine (DMPE), and distearoylphosphatidylcholine (or 1,2-distearoyl-sn-glycero-3-phosphocholine, DSPC). According to embodiments of the present disclosure, the non-cationic lipid comprised in the present

LNP is a helper lipid of distearoylphosphatidylcholine (DSPC).

According to some embodiments of the present disclosure, the cationic lipid and the non-cationic lipid are respectively present in the LNP at a molar ratio of 1:5 to 5:1.

In alterative or optional embodiments, the present LNP further comprises a steroid. Examples of steroid suitable for use in the present LNP include, but are not limited to, cholesterol, lanosterol, ergosterol, phytosterol, stigmasterol, and brassicasterol. In one working example, the present LNP further comprises cholesterol.

Another aspect of the present disclosure is directed to a method for treating a disease in a subject in need thereof, comprising administering an effective amount of the LNP set forth above to the subject.

According to embodiments of the present disclosure, the disease may be a cancer, an infectious disease, or an autoimmune disease.

Examples of the cancer treatable by the present method include, but are not limited to, a bladder cancer, a bone cancer, a bone marrow cancer, a brain cancer, a breast cancer, a cholangiocarcinoma, a colon cancer, an esophagus cancer, a gastrointestinal cancer, a gum cancer, a head and neck cancer, a kidney cancer, liver cancer, a lung cancer, a nasopharyngeal carcinoma, a leukemia, a lymphoma, an ovary cancer, a prostate cancer, a skin cancer, a stomach cancer, a testis cancer, a tongue cancer, and a uterus cancer.

In some preferred embodiments of the present disclosure, when the disease is the lung cancer, the present LNP comprises a cationic lipid selected from the group consisting of

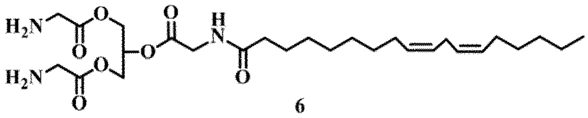

6

,

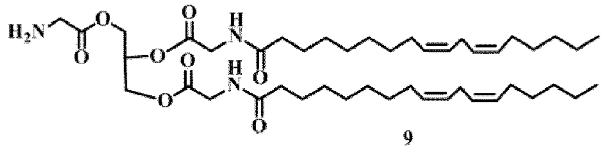

9

,

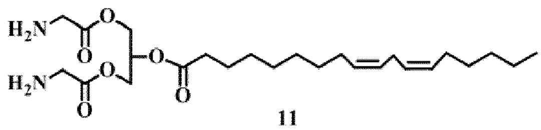

11

,

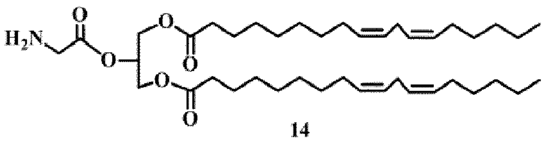

14

,

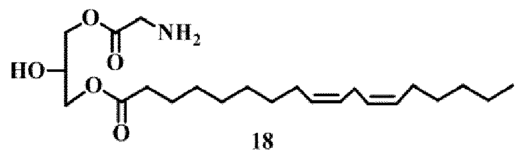

18

,

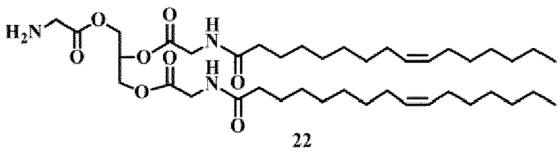

22

,

and

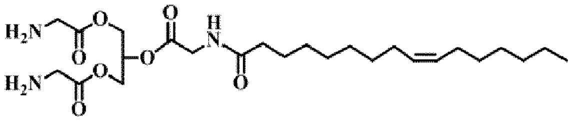

24

;

and the API is a miRNA comprising a sequence of SEQ ID NO:1. Preferably, the miRNA has a sequence of SEQ ID NO:2, 3, 4, or 5.

In other preferred embodiments, when the disease is the lung cancer, the present LNP comprises the cationic lipid of

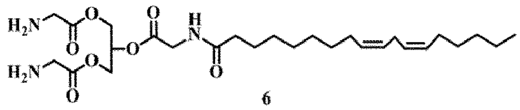

6

or

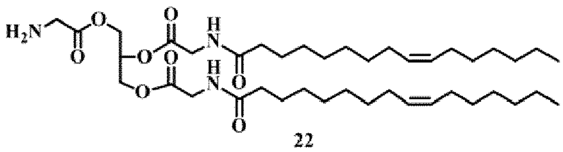

22

;

and the API is a siRNA having a sequence of SEQ ID NO: 6 or 7.

According to some embodiments of the present disclosure, the subject is a human.

Still another aspect of the present disclosure is directed to a method for delivering an API into a cultivated cell, comprising contacting the cultivated cell with an effective amount of the LNP set forth above, wherein the API is a nucleic acid, a polypeptide, a protein, a carbohydrate, a proteoglycan, a glycoprotein, or a combination thereof.

Example of the nucleic acid suitable for use in the present method includes, but is not limited to, mitochondrial DNA (mtDNA), chloroplast DNA (cpDNA), plasmid, messenger RNA (mRNA), small interfering RNA (siRNA), ribosomal RNA (rRNA), transfer RNA (tRNA), microRNA (miRNA), and aptamer. Preferably, the miRNA is a precursor miRNA or a mature miRNA.

Example of the cultivated cell suitable for use in the present method includes, but is not limited to, a multipotent stem cell and an immune cell.

Many of the attendant features and advantages of the present disclosure will becomes better understood with reference to the following detailed description considered in connection with the accompanying drawings.

## BRIEF DESCRIPTION OF THE DRAWINGS

The present description will be better understood from the following detailed description read in light of the accompanying drawings, where:

FIG. 1 are photographs of agarose gel electrophoresis revealing the level of DNA fragments extracted from liposomes composed by the present cationic lipids in accordance with one embodiment of the present disclosure;

FIG. 2A are flow cytometry histograms depicting florescence intensities emitted from A549 cells after co-cultivating with the present lipid nanoparticles (LNPs) containing various concentration of siRNAs in accordance with one embodiment of the present disclosure; and

FIG. 2B are flow cytometry histograms depicting florescence intensities emitted from NCI-H460 cells after co-cultivating with the present lipid nanoparticles (LNPs) containing various concentration of siRNAs in accordance with one embodiment of the present disclosure.

## DESCRIPTION

The detailed description provided below in connection with the appended drawings is intended as a description of the present examples and is not intended to represent the only forms in which the present example may be constructed or utilized. The description sets forth the functions of the example and the sequence of steps for constructing and operating the example. However, the same or equivalent functions and sequences may be accomplished by different examples.

## 1. DEFINITIONS

For convenience, certain terms employed in the specification, examples and appended claims are collected here. Unless defined otherwise, all technical and scientific terms used herein have the same meaning as commonly understood by one of the ordinary skilled in the art to which this invention belongs.

The singular forms "a", "and", and "the" are used herein to include plural referents unless the context clearly dictates otherwise.

The term "alkyl" used herein refers to a straight or branched hydrocarbon group, containing 9-25 carbon atoms. Examples of $C_{9-25}$ alkyl suitable for use in the present invention include nonyl, decyl, undecyl (also hendecyl), dodecyl, tridecyl, tetradecyl, pentadecyl, hexadecyl, heptadecyl, octadecyl, nonadecyl, icosyl, heneicosyl (also henicosyl), docosyl, tricosyl, tetracosyl, and pentacosyl.

The term "alkenyl" used refers to a long, straight hydrocarbon group, containing 13-21 carbon atoms and one or more double bonds. Examples of $C_{13-21}$ alkenyl suitable for used in the present invention include tridecenyl, tetradecenyl, pentadecenyl, hexadecenyl, heptadecenyl, octadecenyl, nonadecenyl, icosenyl, and heneicosenyl (also henicosenyl). Generally, the total numbers of carbon atoms (C) and double bonds (D) in alkenes are respectively represented by numerical symbol of "C:D", such as 14:1, 16:1, 18:1, 18:2, 18:3, 20:4, or 20:5.

The term "active pharmaceutical ingredient (API)" used in this paper refers to an ingredient that is biologically active as a, or a part of the form of medicine, drugs, compositions, vaccines, and/or pharmaceutical products, by which a disease and/or a condition in a subject may be delayed, ameliorated, hindered or prevented. Examples of APIs suitable for use in the present disclosure include, but are not limited to, nucleic acids, polypeptides, proteins, carbohydrates, proteoglycans, glycoproteins, and a combination thereof.

The term "cationic lipid" as used herein refers to those lipids having one or more fatty acid or fatty alkyl chains and attaining positive charge(s) through one or more amines present in the polar head group. The cationic lipid is typically protonated (*i.e.*, positively charged) at a pH below its pKa.

The term "non-cationic lipid" as used herein refers to lipids that do not possess positive charge(s), such as a neutral lipid or an anionic lipid. According to embodiments of the present disclosure, the term "non-cationic lipid" also intend to encompass "helper lipid" known to stabilize the structure of LNP, which in turn enhance the delivery of API carried by the LNPs A helper lipid also helps optimizing the encapsulation of APIs (*e.g.*, nucleic acids), protect them from degradation, and facilitate their transport into target cells. Examples of the helper lipid suitable for use in the present disclosure include, but are not limited to, dioleoylphosphatidylethanolamine (DOPE), dipalmitoylphosphatidylcholine (DPPC), distearoylphosphatidylethanolamine (DSPE), dimyristoylphosphatidylethanolamine (DMPE), and distearoylphosphatidylcholine (or 1,2-distearoyl-sn-glycero-3-phosphocholine, DSPC).

The term "steroid" as used herein refers to a class of chemicals that derived from a core structure (*i.e.*, steroid nucleus) of gonane, or cyclopentanoperhydrophenanthrene, which is a tetracyclic hydrocarbon with no double bonds. Examples of steroid that play roles in the formation of the present LNPs include, but are not limited to, cholesterol, lanosterol, ergosterol, phytosterol, stigmasterol, and brassicasterol.

The term "microRNA" or "miRNA" used herein refers to a class of non-coding RNAs that play roles in regulating gene expression. Most miRNAs are transcribed from DNA sequences into primary miRNAs (pri-miRNAs) and processed into precursor miRNAs (pre-miRNAs) and finally mature miRNAs. Accordingly, the term miRNA used herein refers to all non-coding RNAs involved in miRNA processing and maturation, preferably including precursor miRNAs and mature miRNAs.

The term "infectious diseases" used herein refers to disorders caused by pathogens, such as bacteria, viruses, fungi or parasites, which typically cause acute symptoms, such as fever, inflammation, upper respiratory symptoms, diarrhea, and the like.

The term "subject" or "patient" is used interchangeably herein and is intended to mean a mammal including the human species that is treatable by the API within the present cationic lipids. The term "mammal" refers to all members of the class Mammalia, including humans, primates (*e.g.*, monkey, and chimpanzee), domestic and farm animals, such as rabbit, pig, goat, sheep, and cattle; as well as zoo, sports or pet animals (*e.g.*, a horse, a dog, a cat and etc); and rodents, such as mouse, rat, guinea pig, and hamster. In a working example, the subject is a human. Further, the term "subject" or "patient" intended to refer to both the male and female gender unless one gender is specifically indicated.

## 2. DETAIL DESCRIPTION OF PREFERRED EMBODIMENTS

The present disclosure is based, at least in part, on the discovery that some novel cationic lipids may act as vehicles for the delivery of polynucleotides into cells. Accordingly, the present disclosure provides a novel cationic lipid and a composition thereof that serve as a vehicle for the delivery of therapeutic agents. Also disclosed herein is a treatment of diseases by use of the present cationic lipid and/or composition.

## 2.1 Cationic lipids of the present disclosure

The present disclosure aims at providing novel cationic lipids and compositions comprising the same for delivering therapeutic agents. In one aspect, the present invention relates to a cationic lipid of formula (I),

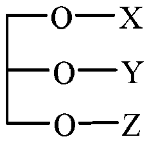
(I),

wherein, at least one of X and Y is $-(C{=}O)CH_2NH_2$, and the other X, Y, and Z are independently selected from the group consisting of $-(C{=}O)CH_2NH_2$, $-[(C{=}O)CH_2NH](C{=}O)R^1$, $-(C{=}O)R^1$, and H, wherein $R^1$ is $C_{9-25}$ alkyl, or $C_{13-21}$ alkenyl.

In some embodiments, $R^1$ is $C_{9-25}$ alkyl; in preferred embodiments, $R^1$ is $C_{11-23}$ alkyl; in other preferred embodiments, $R^1$ is $C_{13-21}$ alkyl; and in still other preferred embodiments, $R^1$ is $C_{15-19}$ alkyl. In some working examples, $R^1$ is $C_{15}$ alkyl.

In other embodiments, $R^1$ is $C_{13-21}$ alkenyl; and in preferred embodiments, $R^1$ is $C_{15-19}$ alkenyl. In some working examples, $R^1$ is $C_{15}$ alkenyl; in other working examples, $R^1$ is $C_{17}$ alkenyl.

Exemplary compound of formula (I) is

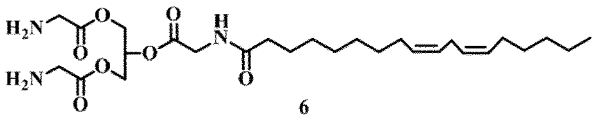
6

(compound 6, TGG-linoleic acid),

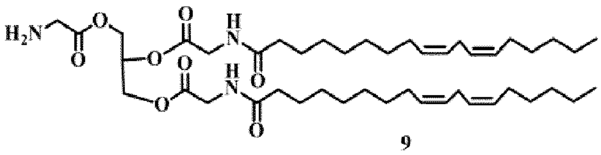
9

(compound 9, TGG-di(linoleic acid),

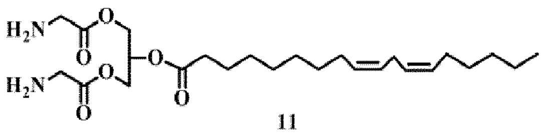
11

(compound 11, DGG-linoleic acid),

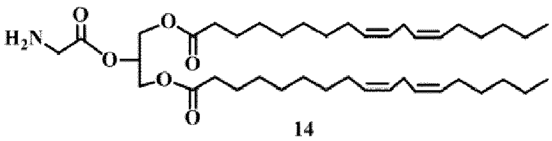
14

(compound 14, MGG-di(linoleic acid),

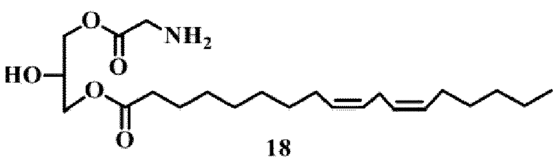
18

(compound 18, MGG-linoleic acid),

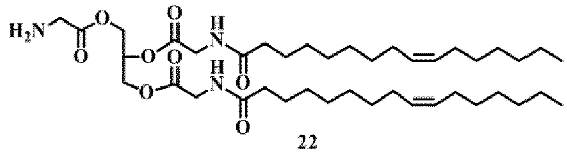

22

(compound 22, TGG-di(palmitoleic acid), or

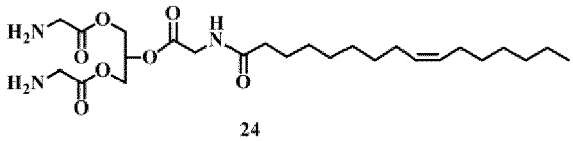

24

(compound 24, TGG-palmitoleic acid).

The cationic lipids of the present disclosure may be prepared in accordance with procedures described in the working examples. Briefly, amino group ($NH_2$) of glycine is firstly protected by protecting groups such as phenacyl (PAc) or tert-butyloxycarbonyl (Boc) groups. Then, at least one PAc- or Boc protected glycine is linked with glycerol via condensation reaction (*i.e.*, dehydration) occurring between at least one hydroxyl group of glycerol and the carboxyl group of glycine. Finally, one or two fatty acid(s) are linked to the rest of hydroxyl group(s) of glycerol to form the present cationic lipid. According to alternative embodiments, a condensation reaction occurs between the carboxyl group of fatty acid and the amino group of glycine gives rise to an amide bond therebetween, thereby produces an intermediate compound having a free carboxyl group for subsequent reacting with hydroxyl groups of glycerol, thus produces the final cationic lipid products of the present invention. The detail reaction conditions and chemical agents for producing the present cationic lipid compounds can be modified by the skill persons in the art without departing from the spirit of the present disclosure.

Examples of fatty acid suitable for use in production of the present compound of formula (I) include, but are not limited to, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid, and docosahexaenoic acid. In working examples, the fatty acid used in the production of the present compound of formula (I) is palmitoleic acid or linoleic acid.

All stereoisomers of the present compounds, such as those which may exist due to asymmetric carbons on each X, Y, and Z substituents of the compound of formula (I) including enantiomeric and diastereomeric forms, are contemplated within the scope of this invention.

### 2.2 Lipid nanoparticles

Also disclosed herein is a novel lipid nanoparticle (LNP) that facilitates the intracellular delivery of biologically active and therapeutic molecules into cells of patients.

According to embodiments of the present disclosure, the lipid nanoparticle (LNP) comprises a cationic lipid of formula (I) described above, a non-cationic lipid, and an active pharmaceutical ingredient (API). Examples of the non-cationic lipids suitable for constructing the present LNP include, but are not limited to, helper lipids, phospholipids, PEGylated lipids, PEGylated phospholipids, and a combination thereof. Examples of the helper lipid suitable for use in the present disclosure include, but are not limited to, dioleoylphosphatidylethanolamine (DOPE), dipalmitoylphosphatidylcholine (DPPC), distearoylphosphatidylethanolamine (DSPE), dimyristoylphosphatidylethanolamine (DMPE), and distearoylphosphatidylcholine (or 1,2-distearoyl-sn-glycero-3-phosphocholine, DSPC). According to embodiments of the present disclosure, the non-cationic lipid comprised in the present LNP is a helper lipid of distearoylphosphatidylcholine (DSPC). Examples of PEGylated lipids suitable for use in the present disclosure include, but are not limited to, PEG conjugated to diethylene glycol (DEG-PEG) (*e.g.*, 1,2-dimyristoyl-rac-glycero-3-methoxypolyethylene glycol-2000 (DEG-PEG 2000); PEG conjugated to dialkyloxypropyl (PEG-DAA) (*e.g.*, dioleoylphosphatidylethanolamine (DOPE)); PEG conjugated to diacylglycerol (PEG-DAG) (*e.g.*, 1,2-dioleoyl-sn-glycero-3-phosphoethanolamine-N-[methoxy(polyethylene glycol)-2000] (DOPE-PEG 2000); PEG coupled to phosphatidylethanolamine (PEG-PE) (*e.g.*, 1,2-distearoyl-sn-glycero-3-phosphoethanolamine-N-[methoxy(Polyethylene Glycol)-2000], DSPE-PEG 2000); and a combination thereof. In one working example, the PEGylated lipid is DEG-PEG 2000.

According to embodiments of the present disclosure, the present cationic lipids and the non-cationic lipid are present in the LNP at a molar ratio of 1:5 to 5:1, such as 1:5, 1:4.5, 1:4, 1:3.5, 1:3, 1:2.5, 1:2, 1:1.5, 1:1, 1.5:1, 2:1, 2.5:1, 3:1, 3.5:1, 4:1, 4.5:1 or 5:1. In some working examples, the present cationic lipid and the non-cationic lipid are present

in the LNP at a molar ratio of 1:1. In alternative working examples, the present cationic lipid and the non-cationic lipid are present in the LNP at a molar ratio of about 4.3:1.

Alternatively or in addition, the present LNP may further comprise a steroid to help stabilize the structure of the LNP. Examples of the steroid suitable for constructing the present LNP include, but are not limited to, cholesterol, lanosterol, ergosterol, phytosterol, stigmasterol, and brassicasterol. Preferably, the present LNP further comprises cholesterol.

According to some embodiments of the present disclosure, each LNP has a hydrophilic core enclosed by a lipid bilayer consisting of both cationic (*e.g.*, at least one compounds of formula (I)) and non-cationic lipids, and optionally, the steroid and/or the helper lipids embedded within the lipid bilayer; accordingly, APIs that are hydrophilic in nature may be disposed in the hydrophilic core, while APIs that are hydrophobic in nature are intercalated between the hydrophobic region between the lipid bilayer. In other embodiments, instead of having a hydrophilic core enclosed by a lipid bilayer as indicated above, each LNP is in the structure of a micelle with a plurality of smaller inverse micelles enclosed therein. The micelle has a hydrophobic core enclosed by a single layer of lipids (*e.g.*, the cationic and non-cationic lipids of the present disclosure) with the hydrophobic tails of the lipids extending toward the center, while the hydrophilic head groups of the lipids are facing out. Additionally, multiple inverse micelles that are smaller than the micelle in size may be enclosed within the hydrophobic core of the micelle; note that the term "inverse micelle" refers to a micelle, in which the head groups of the lipids are at the center while the hydrophobic tails of the lips are extending out. Accordingly, the hydrophilic API (*e.g.*, nucleic acids) may be disposed inside the hydrophilic cores of the inverse micelles.

The present LNP can be produced via procedures and/or tools well known in the art, including thin-layer hydration, ethanol injection, microfluidic devices, and the like, in which the cationic lipids (*i.e.*, the compounds of formula (I)), the non-cationic lipids, and optionally, the steroid, are self-assembled to form a lipid bilayer, and the APIs may be directly encapsulated within the LNP, or may be coupled to the cationic lipids before being encapsulated in the LNP, thereby producing a complex in the form of nanoparticles, liposomes, or micelles. The APIs to be delivered by the present LNP, either in the form of a liquid or a solid, may be a nucleic acid, a polypeptide, a protein, an antibody, a carbohydrate, a proteoglycan, a glycoprotein, a small molecule, or a combination thereof.

In some embodiments of the present disclosure, the API is the nucleic acid, which includes but is not limited to, mitochondrial DNA (mtDNA), chloroplast DNA (cpDNA), plasmid, messenger RNA (mRNA), small interfering RNA (siRNA), ribosomal RNA (rRNA), transfer RNA (tRNA), microRNA (miRNA), or aptamer. In preferred embodiments, the API is miRNA or siRNA. Examples of miRNA includes, but is not limited to, a precursor miRNA or a mature miRNA. In some working examples, the miRNA to be encapsulated and delivered via the present cationic lipids comprising a sequence of SEQ ID NO: 1; more preferably, the miRNA has a sequence of SEQ ID NO:2, 3, 4, or 5. In other working examples, the siRNA used to be delivered via the present cationic lipids has a sequence of SEQ ID NO: 6 or 7. In some alternative embodiments, the API is a plasmid.

### 2.3 Treatments via use of the present cationic lipids

The cationic lipids of formula (I) and lipid nanoparticles thereof are useful for the delivery of biologically active agents, thus they may be used in the treatment of diseases. The present disclosure thus encompasses a method of treating a disease in a subject in need thereof. The method mainly includes, administering an effective amount of the composition (*i.e.*, the lipid nanoparticles (LNPs) composed of the compound of formula (I)) to the subject.

The present lipid nanoparticles comprising the cationic lipids of formula (I) may be formulated into powders, granules, solutions or suspensions, suppositories or patches, and such formulations can be administered or parenterally, to effectively transport the active ingredients to the appropriate or desired site of action and lesions. According to the present disclosure, the present lipid nanoparticle can be parenterally administered to lesions *(e.g.*, the tumor) of the subject. Exemplary suitable parenteral route includes, but is not limited to, transdermal, percutaneous, intravenous, intramuscular, intra-arterial, intramedullary, intrathecal, intraventricular, intradermal, subcutaneous, rectal, intravaginal, and intraperitoneal routes. Suitable routes vary, as recognized by those skilled in the art, depending on the particular condition being treated, the severity of the condition, the individual patient parameters including age, physical condition, size, gender and weight, the duration of the treatment, the nature of concurrent therapy (if any), the dosage and the nature of active ingredients, genetic factors and like factors within the knowledge and expertise of the health practitioner. These factors are well known to those of ordinary skill in the art and can be addressed with no more than routine experimentation. In general, the most appropriate route of administration depends on a variety of factors including the agent's stability in the environment of the circulatory system, and/or the condition of the subject (*e.g.*, the severity of lung cancer of the subject, or whether the subject is able to tolerate conventional treatment). According to some embodiments of the present disclosure, the present lipid nanoparticle is formulated into injectable formulation for parental administration. According to other embodiments of the present disclosure, the present lipid nanoparticle is formulated into patches for transdermal administration.

The present lipid nanoparticle can be administered at a frequency that effectively prevent, inhibit, suppress, or

treat diseases, conditions, or traits in the subject. In some embodiments, the lipid nanoparticle can be administered at a frequency of four times a day to once every three months; for example, at a frequency of four times a day, three times a day, twice a day, once a day, once every other day, once every third day, once every week, once every other week, once monthly, twice monthly, thrice monthly, once every other month, or once every three months. Preferably, the lipid nanoparticle is administered to the subject at a frequency of twice per week (once every third or four day). Optionally, the lipid nanoparticle is administered to the subject at a frequency of once a week. Still optionally, the lipid nanoparticle is administered to the subject at a frequency of once every other week.

According to embodiments of the present disclosure, diseases treatable by the present method may be cancers, infectious diseases (including inflammatory diseases), transplant and/or tissue rejection, autoimmune diseases, and the like.

Examples of cancer treatable by the present method include, but are not limited to, bladder cancer, bone cancer, bone marrow cancer, brain cancer, breast cancer, cholangiocarcinoma, colon cancer, esophagus cancer, gastrointestinal cancer, gum cancer, head and neck cancer, kidney cancer, liver cancer, lung cancer, nasopharyngeal carcinoma, leukemia, lymphoma, ovary cancer, prostate cancer, skin cancer, stomach cancer, testis cancer, tongue cancer, and uterus cancer.

In one preferred embodiment, the lung cancer subject is treated via administering to the subject an effective amount of the present lipid nanoparticle, which comprises the cationic lipid of

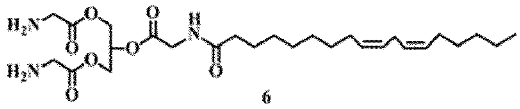

6

or

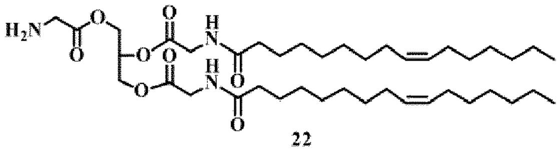

22

,

and the siRNA having a sequence of SEQ ID NO: 6 or 7.

In another preferred embodiment, the lung cancer subject is treated administering to the subject an effective amount of the present lipid nanoparticle, in which the cationic lipid is selected from the group consisting of

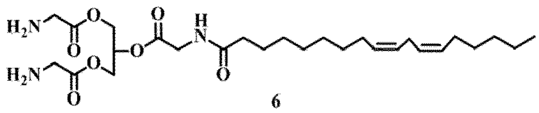

6

,

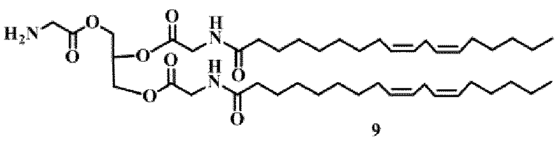

9

,

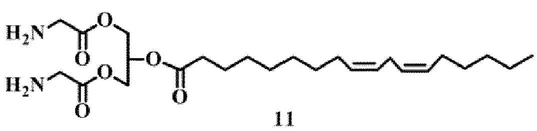

11

,

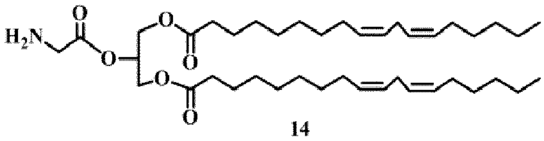

14

,

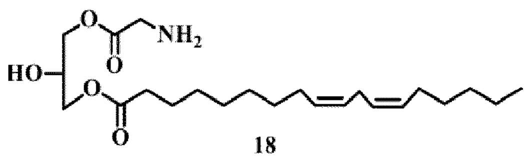

18

,

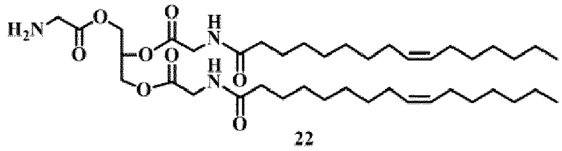

22

,

and

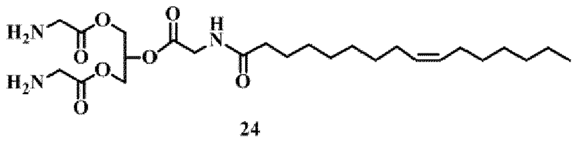

24

;

and the miRNA has a sequence selected from the group consisting of SEQ ID NO:2, 3, 4, and 5.

**2.4 API Delivery platform via use of the present cationic lipids**

The present lipid nanoparticles (LNPs) are useful for the delivery of APIs into cultivated cells, thus they may be used as a platform for the delivery of therapeutic materials in preparation of cellular therapies. The present disclosure thus encompasses a method of delivering an API into a cultivated cell. The method mainly includes, contacting the cultivated cell with an effective amount of the present LNP, wherein the API is a nucleic acid, a polypeptide, a protein, a carbohydrate, a proteoglycan, a glycoprotein, or a combination thereof.

The cultivated cells provided in the present embodiments may be isolated or derived from subjects, and potent to differentiate or develop into cellular transplants with therapeutic benefits by receiving genes or gene products delivered via the present LNP, such that the cellular transplants are useful in and beneficial to cell therapy adaptive to select fields, including regenerative medicine, immune system disorders, and cancer. Generally, the cultivated cells include stem cells and immune cells. Examples of stem cells suitable for use in the present method include, but are not limited to pluripotent stem cells (PSCs; for example, embryonic stem cells (ESCs), epiblast stem cells (EpiSCs), embryonic germ cells (EGCs), and induced pluripotent stem cells (iPSCs)), adult stem cells (ASCs; for example, hematopoietic stem cells (HSCs), skin stem cells (SSCs), neural stem cells (NSCs), and mesenchymal stem cells (MSCs)), and cancer stem cells (CSCs). Examples of immune cells suitable for use in the present method include but are not limited to, T cells, dendritic cells (DCs), natural killer (NK) cells, and macrophages. The immune cells express genetically engineered antigen receptors, including engineered T cell receptors (TCRs) and functional non-TCR antigen receptors, such as chimeric antigen receptors (CARs), including activating, stimulatory, and costimulatory CARs, and combinations thereof.

By the virtue of the above features, the present disclosure provides novel cationic lipids capable of encapsulating and delivering polynucleotides loaded therein to cells, thereby allowing diseases including cancers to be effectively and efficiently treated.

**EXAMPLES**

**Materials and Methods**

*Cell culture*

NCI-H460 cells (large cell lung cancer cell line) were cultured in Roswell Park Memorial Institute (RPMI) 1640 medium supplemented with heat-inactivated 10% fetal bovine serum (FBS) and 1 % penicillin-streptomycin (PS) solution. A549 cells (established from human cancerous lung tissues) were cultured in Ham's F-12K (Kaighn's) medium supplemented with heat-inactivated 10% FBS and 1 % PS solution. All cells were cultured at 37°C under an atmosphere containing 5% $CO_2$.

*Plasmid stabilization via the present cationic lipids*

The present cationic lipids (*i.e.*, compounds 6, 9, 11, 14, 18, 22, or 24), lecithin, and cholesterol were fully mixed at a molar ratio of 2:2:1 and dissolved in ethanol completely, and the solution was diluted from 30 to 50 μL by DPBS

buffer. The diluted solution was then co-cultivated with 50 μL of sucrose aqueous (5% w/v) containing 1.5 μg of EGFP-plasmid (pUC19, 4564bp, Addgene) at room temperature for one hour to produce a cationic lipid-DNA complex. The obtained solution was divided into two vials, one of which was added with 1 μL of DNase and 10 μL of 10 × DNase buffer and incubated at 37 °C for further 15 minutes. After that, 100 μL of phenol/chloroform (5/1) was added and mixed by centrifugation at 13000 × g for 5 minutes to extract DNA. All the samples were subjected to gel electrophoresis (1% agarose gel, 110V).

*Preparation of cationic lipid-RNA complexes*

To produce the present composition, the present cationic lipids (compound 6 or 22), cholesterol, DEG-PEG 2000, and distearoylphosphatidylcholine (DSPC) were fully mixed in ethanol at a molar ratio of 50:38.5:1.5:10, and the total volume was brought to 500 μL, thereby producing a liquid formulation of lipid nanoparticles (LNPs). As for control group, D-lin-MC3-DMA (MC30), an ionizable amino lipid that conventionally used in LNPs preparation, was mixed with cholesterol, DEG-PEG 2000, and DSPC at the same molar ratio as the present LNPs. 0.268 mM, 265 μL of miRNAs or siRNAs (*e.g.*, the polynucleotides of SEQ ID NO: 2 to 7) were added in 695 μL acidic buffer (composed of 100 mM citrate buffer (pH4.0) and nuclease-free water) to produce a RNA working solution, and the total volume was brought to 960 μL. The RNA working solution and the LNPs (molar ratio was 1:6) were respectively pumped into the two inlets and mixed in the channels of the microfluidics device (NanoAssemblr platform) with flow rate ratio (FRR) set to 3 and total flow rate (TFR) set to 12 mL/min, and the confluent liquid reaching the outlet of the microfluidics was collected with a total volume of 1.2 mL. The collected mixture was then filtrated and centrifuged at 2,000 g for 20 minutes × three times by centrifugal filters (membrane filter with a cutoff molecular weight of 10 kDa), and the filtrate containing lipid nanoparticles having miRNA or siRNA encapsulated therein *(i.e.,* cationic lipid-RNA complexes) were stored at 4 °C until use.

*Cationic lipid-RNA complexes characterization*

Particle sizes and zeta potentials of cationic lipid-RNA complexes were respectively determined by using a particle size and zeta potential analyzer (DelsaNano C, Beckman Coulter).

The encapsulation ratio of liposomes for encapsulating miRNAs or siRNAs was determined by fluorescence reader (Infinite F200 Pro) with the aid of RNA quantification kit containing a fluorescent dye (RiboGreen). Absorption spectra of miRNAs or siRNAs with known concentrations (*i.e.* 100 μg/mL) were first acquired for the construction of a standard curve. The cationic lipid-RNA complexes (typically with a total volume of 50 μL for each sample well) were then mixed with the reagent from the RNA quantification kit in each well. Optionally, the cationic lipid-RNA complexes were subjected to lysis buffer, thereby bringing RNA back into suspension. The amount of miRNAs or siRNAs were quantified by interpolating their absorption strengths at 535 nm to that of the standard curve, and the encapsulation efficiency (EE) was calculated by the following formula:

$$\text{Encapsulation efficiency (EE \%)} = (\text{Wt/Wi}) \times 100\ \%,$$

where Wt is the total amount of miRNAs or siRNAs released in the LNPs lysis suspension, and Wi is the total quantity of miRNAs or siRNAs added initially during preparation.

*Flow cytometry analysis*

Cell sorting was conducted via flow cytometry with fluorochrome-conjugated miRNAs or siRNAs, specifically the miRNAs were conjugated with a far-red-fluorescent label Cy5 dye. After a final wash prior to sorting, cells were filtered through a 35 μm nylon cell strainer for removal of cell clumps, and then subjected to sorting using a flow cytometer (BD LSRFortessa X-20) and analysis software (FlowJo, BD Biosciences).

**Example 1 Chemical synthesis of the present cationic lipids**

The present cationic lipids were respectively synthesized in accordance with steps as described in schemes I-IV. In general, the amino group ($NH_2$) of glycine was firstly protected by phenacyl (PAc) or tert-butyloxycarbonyl (Boc) groups. After the condensation reaction (*i.e.*, dehydration) between glycerol and glycine, and/or between glycerol and fatty acids, the protective groups were removed and reduced to $NH_2$ group.

Scheme I

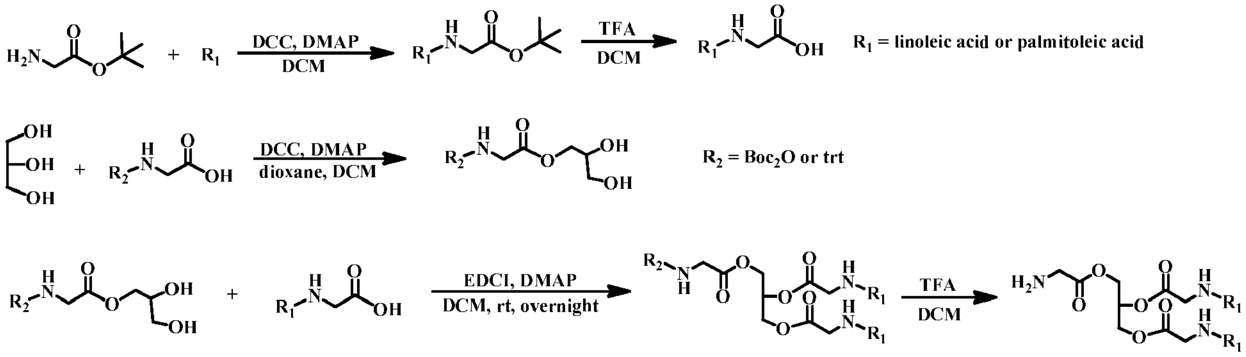


Scheme II

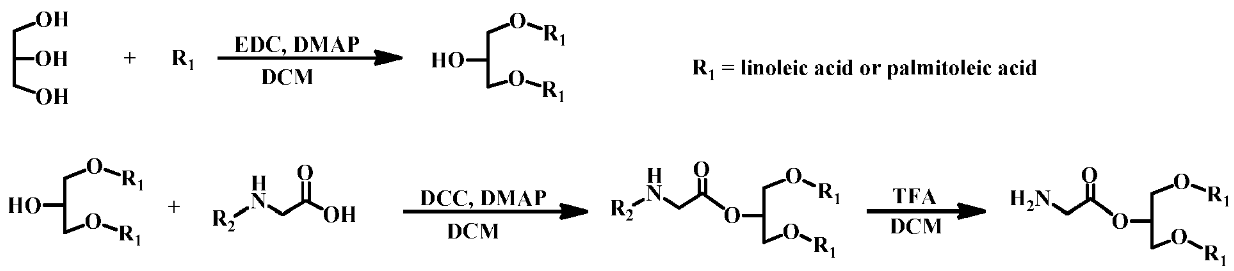


Scheme III

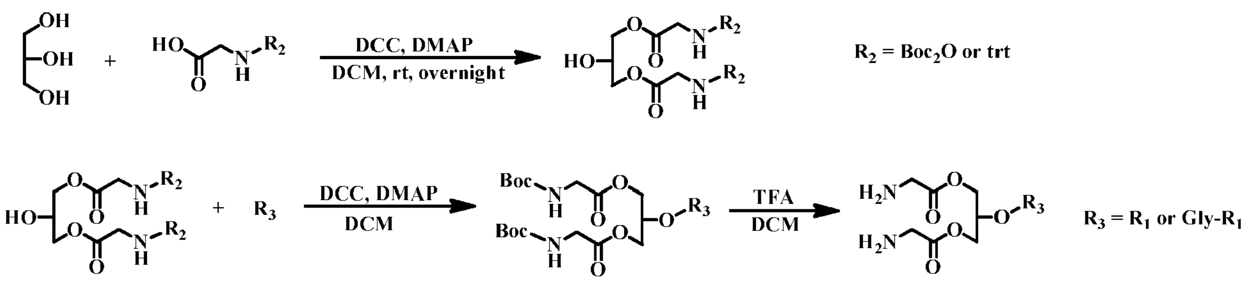


Scheme IV

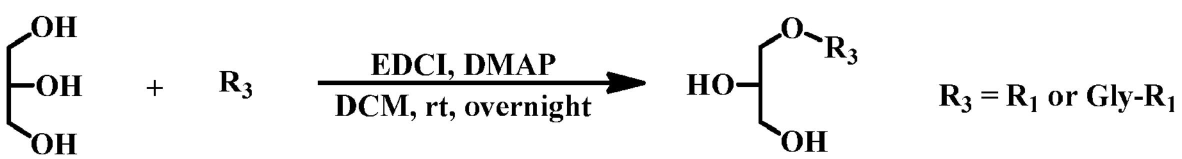


The methods for preparing the exemplary compounds, as well as the analytical data for the compounds thus prepared, are set forth below:

### 1.1 TGG-linoleic acid (Compound 6)

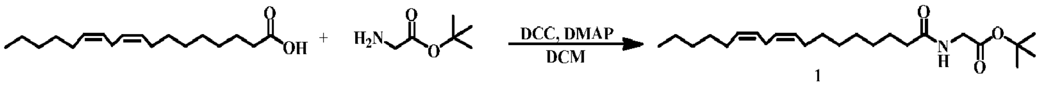


1

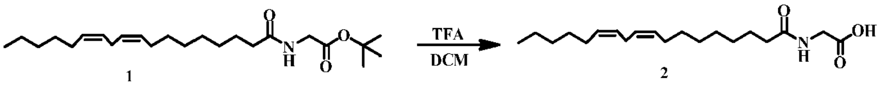


1 2

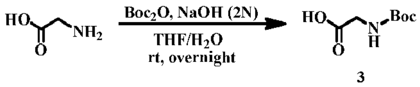


3

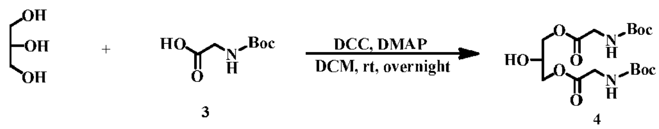


3 4

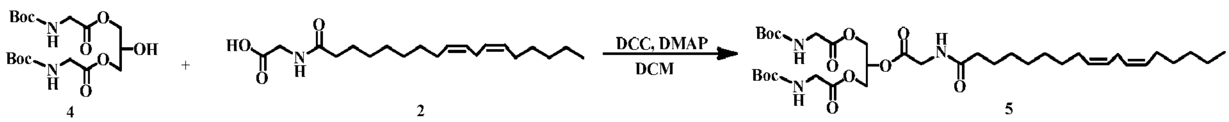


4 2 5

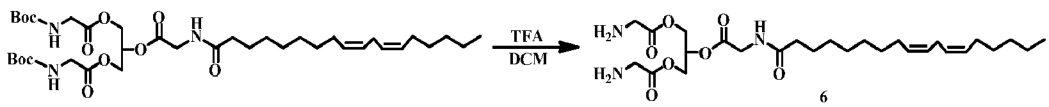


6

***Preparation of tert-butyl 2-((9Z,12Z)-octadeca-9,12-dienamido)acetate (1)*** To a solution of N,N'-Dicyclohexylcarbodiimide (DCC, 0.40 g, 1.96 mmol) dissolved in 10 mL of dichloromethane (DCM), linoleic acid (0.5 g, 1.78 mmol), t-butyl 2-aminoacetate (0.27 mL, 1.96 mmol), and DMAP (0.13, 1.07 mmol) was added. The solution was stirred overnight at room temperature. After fully mixed, 1,3-Dicyclohexyl urea (DCU) was filtered off, the solution was removed by rotary evaporator. The residual solution was purified by column chromatography eluted with ethyl acetare (Hexane:EA, 3:1) to obtain 480 mg of solid (**compound 1**) (yield 69%). $^1$H NMR (600 MHz, $CDCl_3$) δ 5.93 (s, 1H), 5.44 - 5.26 (m, 4H), 3.95 (d, *J* = 4.9 Hz, 2H), 2.78 (t, *J*= 6.9 Hz, 2H), 2.29 - 2.17 (m, 2H), 2.10 - 2.02 (m, 4H), 1.66 (dd, *J* = 15.7, 8.8 Hz, 3H), 1.49 (s, 9H), 1.39 - 1.28 (m, 14H), 0.91 (t, *J* = 7.0 Hz, 3H).

***Preparation of 2-((9Z,12Z)-octadeca-9,12-dienamido)acetic acid (2)*** Trifluoroacetic acid (TFA, 2.80 mL, 36.59 mmol) was lowly added to a solution of **compound 1** (480 mg, 1.22 mmol, dissolved in 8 mL of DCM). The solution was stirred and monitored by TLC. After fully reacted, DCM was removed under reduced pressure. The residue was washed by hexane and EA for several times. Remained solvent was removed by rotary evaporator to obtain **compound 2** for further use.

***Preparation of 2-((tert-butoxycarbonyl)amino)acetic acid (3)*** To a bottle having solution of 2N NaOH (8.66 mL, 17.32 mmol), glycine (1 g, 13.32 mmol) was added, and the mixture was stirred until all glycine was dissolved, then a solution of $Boc_2O$ (3.34 g, 15.32 mmol) dissolved in THF (20 mL) was added into bottle and stirred at room temperature overnight. THF was removed by evaporation. To make basic solution, 2N NaOH solution was added to the residue and washed by EA. The pH value was adjusted to 3-4 by adding 2M HCl. The aqueous phase was extracted with EA. Organic phase was dried over $MgSO_4$, filtered and the solvent was removed by rotary evaporator to obtain 1.38 g of white solid product (**compound 3**, yield 59%). 1HNMR (600 MHz, CDCl3) δ 5.03 (s, 1H), 3.97 (t, J = 20.4 Hz, 2H), 1.48 (s, 9H).

***Preparation of 2-hydroxypropane-1,3-diyl bis(2-((tert-butoxycarbonyl)amino)acetate) (4)*** To a solution of DCC (0.99 g, 4.78 mmol) dissolved in DCM, glycerol (0.2 g, 2.17 mmol), **compound 3** (0.80 g, 4.56 mmol) and DMAP (0.27 g, 2.17 mmol) were added and stirred overnight at room temperature. The mixture was subjected to filtration to remove DCU and then evaporated to give a crude product. Crude product was purified by column chromatography (Hexane:EA, 50:50→30:70) to obtain 130 mg of clear sticky liquid product (**compound 4**, yield 15%). LC-MS (ESI); m/z: $[M+H]^+$ Calcd. for $C_{17}H_{30}N_2O_9$,406.20. Found 407.41.

***Preparation of 2-(2-((9Z,12Z)-octadeca-9,12-dienamido)acetoxy)propane-1,3-diyl bis(2-((tert-butoxycarbonyl)anuno)acetate) (5)*** To a solution of DCC (0.073 g, 0.35 mmol) dissolved in DCM, **compound 4** (0.13 g, 0.32 mmol), **compound 2** (0.12g, 0.35 mmol) and DMAP (0.039 g, 0.32 mmol) were added. The solution was stirred overnight at room temperature. After fully mixed, the solution was filtered to remove DCU, and then evaporated under reduced

pressure to remove DCM. The residual solution was purified by column chromatography (Hexane:EA, 2:3) to obtain 200 mg of white solid product (**compound 5,** yield 86%). $^1$H NMR (600 MHz, CDCl3) δ 5.36 (ddd, J = 27.2, 14.1, 6.3 Hz, 4H), 5.21 (s, 1H), 4.40 (dt, J = 18.0, 9.0 Hz, 2H), 4.34 - 4.23 (m, 2H), 4.13 - 3.97 (m, 2H), 3.97 - 3.82 (m, 4H), 2.79 (t, J = 6.8 Hz, 1H), 2.27 (dd, J = 14.3, 6.9 Hz, 2H), 2.11 (d, J = 5.8 Hz, 1H), 2.06 (dd, J = 13.8, 6.8 Hz, 3H), 1.95 (s, 2H), 1.86 (dd, J = 9.8, 4.7 Hz, 2H), 1.78 (d, J = 13.3 Hz, 2H), 1.70 - 1.61 (m, 3H), 1.47 (s, 18H), 1.41 - 1.26 (m, 16H), 0.91 (t, J = 6.6 Hz, 3H). LC-MS (ESI); m/z: $[M+H]^+$ Calcd. for $C_{37}H_{63}N_3O_{11}$, 725.45. Found 726.7.

***Preparation of 2-(2-((9Z,12Z)-octadeca-9,12-dienamido)acetoxy)propane-1,3-diyl bis(2-aminoacetate) (6)*** To a solution of **compound 5** (0.2 g, 0.28 mmol) dissolved in 6 mL of DCM, TFA (0.42 mL, 5.51 mmol) was added slowly. The solution was stirred until the reactant was consumed. The resulting solution was then evaporated under reduced pressure to remove solvent, and washed with ether and DCM. The residual solvent was removed by evaporation to provide 48 mg of clear sticky product (**compound 6**, yield 33%). $^1$H NMR (600 MHz, MeOD) δ 5.53 - 5.44 (m, 1H), 5.41 - 5.28 (m, 4H), 4.60 - 4.37 (m, 4H), 3.94 - 3.84 (m, 6H), 2.79 (t, *J* = 6.7 Hz, 2H), 2.28 (dd, *J* = 9.8, 5.0 Hz, 2H), 2.07 (dd, *J* = 12.2, 6.2 Hz, 4H), 1.67 - 1.55 (m, 2H), 1.41 - 1.27 (m, 15H), 0.92 (t, *J* = 6.8 Hz, 3H). LC-MS (ESI); m/z: $[M+H]^+$ Calcd. for $C_{27}H_{47}N_3O_7$, 525.34. Found 527.38.

**1.2 TGG-di(linoleic acid) Compound 9**

*Procedure 1*

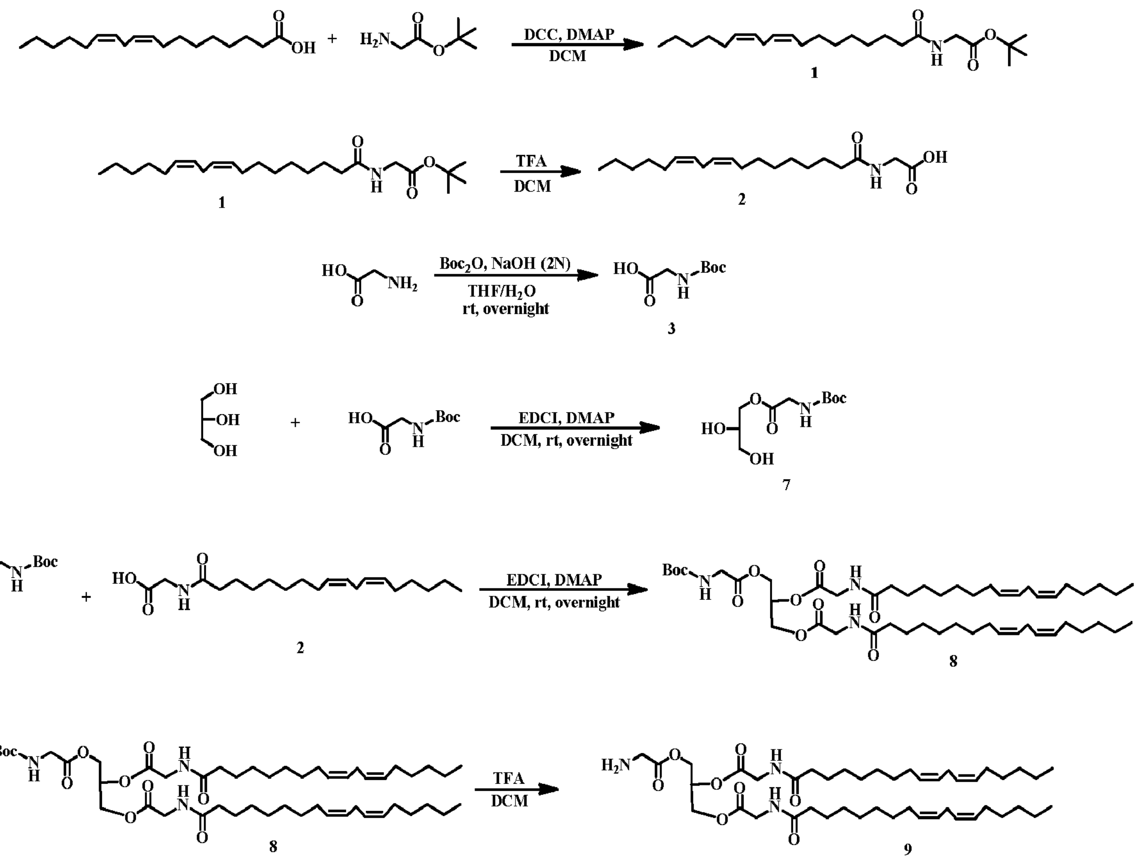


***Preparation of 2,3-dihydroxypropyl 2-((tert-butoxycarbonyl)amino)acetate (7)*** Glycerol (0.42 g, 4.57 mmol), **compound 3** (0.2 g, 1.14 mmol), EDCI (0.35 g, 2.28 mmol) and DMAP (0.14 g, 1.14 mmol) were mixed and dissolved in DCM. The solution was stirred at room temperature overnight. The resulting solution was evaporated to remove DCM to obtain a crude product. The crude product was purified by column chromatography (EA, 100%) to obtain 120 mg sticky liquid product (compound 7, yield 42%). $^1$H NMR (600 MHz, MeOD) δ 4.59 (s, 1H), 4.22 (dd, J = 11.3, 4.3 Hz, 1H), 4.16 - 4.10 (m, 1H), 3.88 - 3.81 (m, 2H), 3.57 (dd, J = 5.6, 2.1 Hz, 2H), 1.46 (s, 9H).

***Preparation of 3-(2-((tert-butoxycarbonyl)amino)acetoxy)propane-1,2-diyl bis(2-((9Z,12Z)-octadeca-9,12-dienamido)acetate) (8)*** **Compound 7** (0.1 g, 0.40 mmol), **compound 2** (0.30 g, 0.88 mmol), EDCI (0.16 g, 1.00

mmol) and DMAP (0.059 g, 0.48 mmol) were mixed and dissolved in DCM. After stirred at room temperature overnight, the mixture was evaporated to remove DCM. The residual solution was extracted with EA/$H_2O$. Organic phase was collected, dried over $MgSO_4$, and filtered. The resulting filtrate was evaporated to remove EA. Crude product was purified by column chromatography (Hexane:EA, 3:7) to obtain 190 mg of brown sticky liquid product (**compound 9**, yield 53%). $^1H$ NMR (600 MHz, CDCl3) δ 5.46 - 5.31 (m, 8H), 4.46 (dd, J = 12.0, 3.5 Hz, 1H), 4.41 - 4.33 (m, 2H), 4.28 (ddd, J = 17.9, 12.1, 6.2 Hz, 1H), 4.14 (q, J = 7.1 Hz, 1H), 4.07 (ddd, J = 19.8, 9.8, 5.1 Hz, 3H), 4.00 (d, J = 3.6 Hz, 2H), 3.96 - 3.83 (m, 2H), 2.79 (t, J = 6.8 Hz, 3H), 2.27 (ddd, J = 9.9, 7.8, 5.2 Hz, 4H), 2.13 - 2.00 (m, 8H), 1.76 - 1.61 (m, 8H), 1.47 (s, 9H), 1.40 - 1.26 (m, 28H), 0.91 (t, J = 7.0 Hz, 6H).

*Procedure 2*

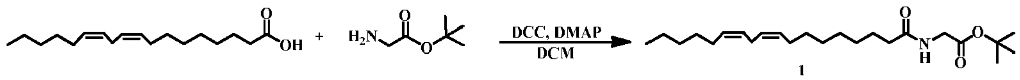


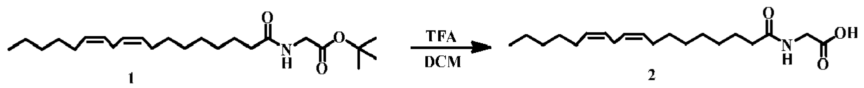


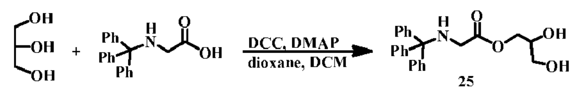


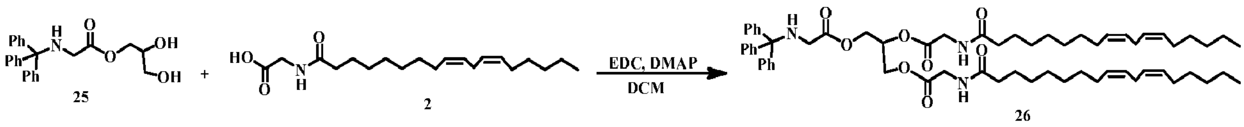


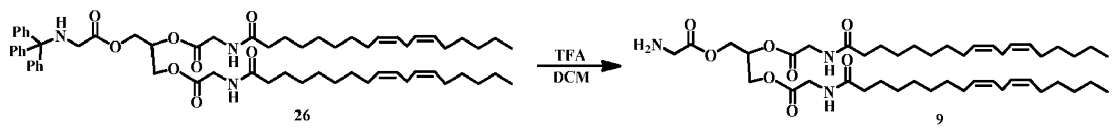


***Preparation of 3-(2-(tritylamino)acetoxy)propane-1,2-diyl bis(2-((9Z,12Z)-octadeca-9,12-dienamido)acetate) (25)*** Glycerol (0.29 g, 3.15 mmol), trt-glycine (0.2 g, 0.63 mmol), were mixed and dissolved in a solvent of DCM/dioxane to provide a mixture. To the mixture, DCC (0.17 g, 0.82 mmol) and DMAP (0.038 g, 0.32 mmol) were gradually added and stirred overnight at room temperature. The solution was filtrated to remove DCU, and evaporated under reduced pressure to remove solvent. Crude product was purified by column chromatography (Hex:EA = 1:3) to obtain 195 mg white solid product (**compound 25**, yield 79%). $^1H$ NMR (600 MHz, MeOD) δ 7.62 - 7.38 (m, 6H), 7.30 (dd, J = 10.6, 5.0 Hz, 6H), 7.24 - 7.16 (m, 3H), 4.11 (dd, J = 11.3, 4.2 Hz, 1H), 4.02 (dd, J = 11.3, 6.4 Hz, 1H), 3.76 (td, J = 9.9, 5.7 Hz, 1H), 3.50 (d, J = 5.7 Hz, 2H), 3.13 (s, 2H).

***Preparation of 3-(2-(tritylamino)acetoxy)propane-1,2-diyl bis(2-((9Z,12Z)-octadeca-9,12-dienamido)acetate) (26)*** **Compound 25** (0.2 g, 0.51 mmol), **compound 2** (0.40 g, 1.18 mmol), EDCI (0.18 g, 1.18 mmol), and DMAP (0.062 g, 0.51 mmol) were mixed and dissolved in DCM. The solution was stirred at room temperature overnight, then evaporated and dried under reduced pressure to remove DCM. Crude product was purified by column chromatography (Hexane:EA, 3:2) to obtain 266 mg of white sticky product (**compound 26**, yield 51%). $^1H$ NMR (600 MHz, $CDCl_3$) δ 7.49 (d, *J* = 7.7 Hz, 6H), 7.36 (d, *J* = 6.4 Hz, 5H), 7.31 (dd, *J* = 6.7, 2.3 Hz, 2H), 5.46 - 5.29 (m, 6H), 5.27 (d, *J* = 6.0 Hz, 1H), 4.26 (ddd, *J* = 19.0, 11.9, 5.4 Hz, 3H), 4.14 (dd, *J* = 11.8, 5.8 Hz, 1H), 4.09 - 3.91 (m, 4H), 2.79 (t, *J* = 6.8 Hz, 3H), 2.27 (dd, *J* = 14.0, 6.6 Hz, 4H), 2.07 (dd, *J* = 12.3, 5.3 Hz, 6H), 1.62 (dd, *J* = 27.0, 19.6 Hz, 11H), 1.52 - 1.20 (m, 28H), 0.97 - 0.81 (m, 6H).

***Preparation of 3-(2-aminoacetoxy)propane-1,2-diyl bis(2-((9Z,12Z)-octadeca-9,12-dienamido)acetate) (9)*** To a solution of **compound 26** (0.27 g, 0.26 mmol) dissolved in DCM, TFA (0.3 mL, 3.93 mmol) was added slowly and stirred until all the reactant was consumed. The solution was evaporated and washed with ether few times to remove solvent. Crude product was purified by column chromatography (Hex:EA = 1:1 → DCM:MeOH, 95:5) to obtain 131 mg

brown gluey product (**compound 9**, yield 64%). $^1$H NMR (600 MHz, MeOD) δ 5.48 - 5.25 (m, 8H), 4.53 - 4.44 (m, 2H), 4.37 (ddd, J = 18.1, 12.1, 5.0 Hz, 2H), 4.06 - 3.81 (m, 6H), 2.79 (t, J = 6.8 Hz, 3H), 2.27 (td, J = 7.5, 4.1 Hz, 4H), 2.08 (dt, J = 10.9, 5.1 Hz, 7H), 1.72 - 1.56 (m, 4H), 1.50 - 1.14 (m, 28H), 0.92 (t, J = 7.0 Hz, 6H). LC-MS (ESI); m/z: $[M+H]^+$ Calcd. for $C_{45}H_{77}N_3O_8$, 787.57. Found 789.48.

**1.3 DGG-linoleic acid (Compound 11)**

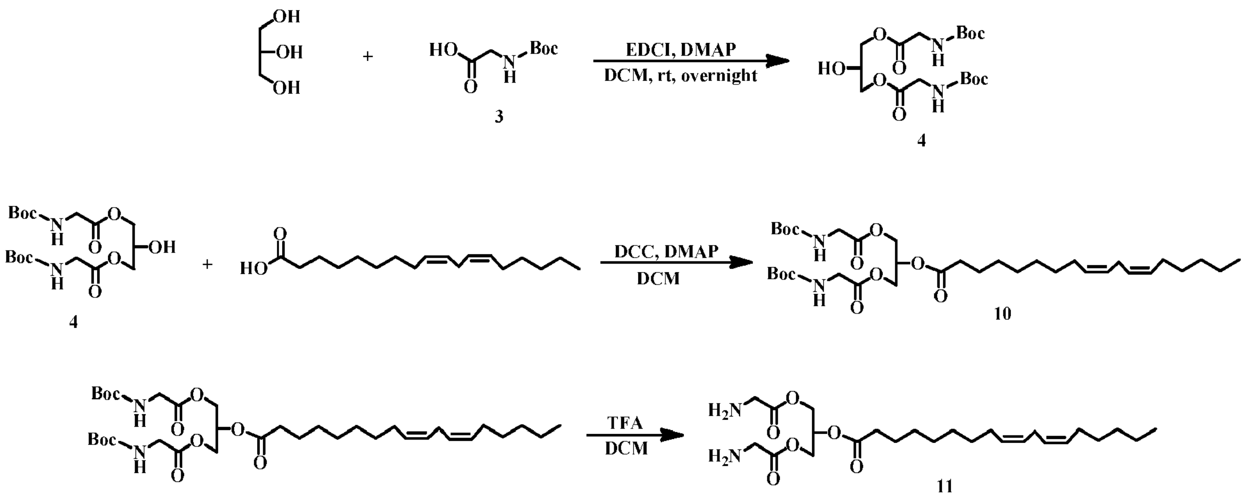


3 4 10 11

***Preparation of 2-((9Z,12Z)-octadeca-9,12-dienoyloxy)propane-1,3-diyl bis(2-((tert-butoxycarbonyl)amino)acetate) (10)*** To a solution of DCC (76 mg, 0.37 mmol) dissolved in 8 mL of DCM, **compound 4** (0.15 g, 0.37 mmol), linoleic acid (93 mg, 0.33 mmo), and DMAP (32 mg, 0.26 mmol) were mixed and added. The mixture was stirred overnight at room temperature, and then filtered to remove DCU. The solution was then evaporated to obtain a crude product. Crude product was purified by column chromatography (Hexane:EA, 7:3) to obtain 140 mg of clear liquid product (**compound 10**, yield 63%). $^1$H NMR (600 MHz, CDCl3) δ 5.33 (ddt, J = 14.9, 10.0, 7.8 Hz, 2H), 5.14 - 5.01 (m, 1H), 4.47 - 4.34 (m, 2H), 4.25 (dd, J = 11.9, 5.7 Hz, 1H), 4.22 - 4.11 (m, 1H), 4.11 - 4.00 (m, 1H), 3.93 (s,4H), 2.79 (t, J = 6.8 Hz, 1H), 2.41 - 2.28 (m, 2H), 2.16 - 2.01 (m, 2H), 1.92 - 1.83 (m, 1H), 1.69 - 1.51 (m, 9H), 1.47 (s, 18H), 1.42 - 1.20 (m, 14H), 0.91 (dd, J = 8.6, 5.3 Hz, 3H).

***Preparation of 2-((9Z,12Z)-octadeca-9,12-dienoyloxy)propane-1,3-diyl bis(2-aminoacetate) (11)*** To a solution containing **compound 10** (0.14 g, 0.21 mmol) dissolved in 6 mL of DCM, TFA (0.40 mL, 5.23 mmol) was added slowly. The solution was stirred until reactant was consumed, and then evaporated to remove solvent and washed with ether and DCM. The resulting was evaporated to obtain **compound 11** (60 mg of clear gluey produce, yield 61%). $^1$H NMR (600 MHz, MeOD) δ 5.51 (s, 1H), 5.45 (dd, J = 9.3, 4.9 Hz, 1H), 5.42 - 5.29 (m, 4H), 4.57 (dt, J = 12.0, 4.1 Hz, 1H), 4.50 - 4.43 (m, 1H), 4.39 (dd, J = 12.0, 6.4 Hz, 1H), 3.96 - 3.82 (m, 3H), 2.79 (t, J = 6.7 Hz, 2), 2.38 (td, J = 7.5, 4.9 Hz, 2H), 2.16 - 1.99 (m, 3H), 1.64 (d, J = 5.9 Hz, 2H), 1.44 - 1.26 (m, 13H), 0.92 (dd, J = 9.4, 4.5 Hz, 3H). LC-MS (ESI); m/z: $[M+H]^+$ Calcd. for $C_{25}H_{44}N_2O_6$, 468.32. Found 470.22.

**1.4 MGG-di(linoleic acid) (Compound 14)**

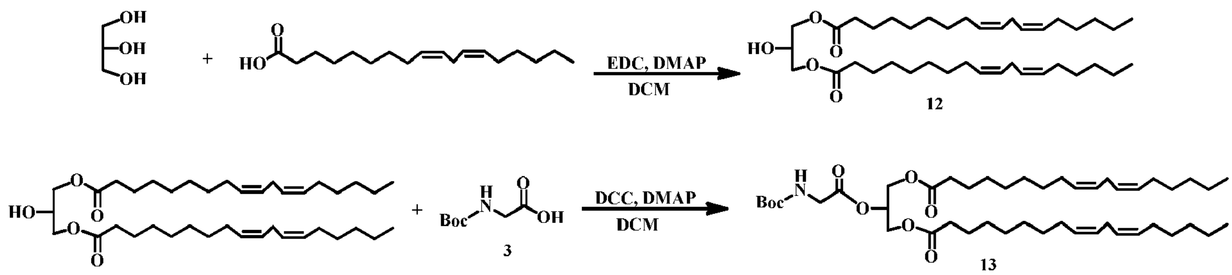


12 3 13

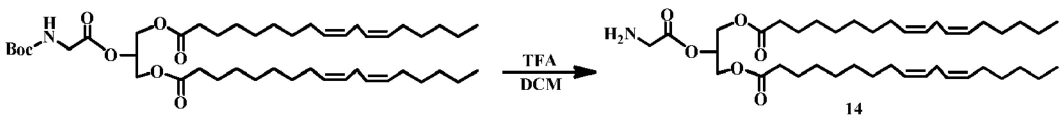


14

***Preparation of (9Z,9'Z,12Z,12'Z)-2-hydroxypropane-1,3-diyl bis(octadeca-9,12-dienoate) (12)*** Glycerol (0.2 g, 2.17 mmol), linoleic acid (1.28 g, 4.56 mmol), EDCI (0.78 g, 5.00 mmol) and DMAP (0.27 g, 2.17 mmol) were mixed dissolved in 12 mL of DCM in a round bottom bottle. The solution was stirred at room temperature overnight, and then evaporated to remove DCM. Crude product was purified by column chromatography (Hexane:EA, 3:2) to obtain 310 mg of product (**compound 12**, yield 23%). $^1$H NMR (600 MHz, CDCl3) δ 5.51 - 5.24 (m, 7H), 4.33 - 3.94 (m, 5H), 2.79 (t, J = 6.8 Hz, 4H), 2.37 (t, J = 7.6 Hz, 4H), 2.07 (q, J = 6.9 Hz, 8H), 1.64 (dd, J = 14.5, 7.2 Hz, 4H), 1.48 - 1.19 (m, 28H), 0.91 (t, J = 7.0 Hz, 6H).

***Preparation of (9Z,9'Z,12Z,12'Z)-2-(2-((tert-butoxycarbonyl)amino)acetoxy)propane-1,3-diyl bis(octadeca-9,12-dienoate) (13)*** To a solution of DCC (0.12 g, 0.60 mmol) dissolved in 10 mL of DCM, **compound 12** (0.31 g, 0.50 mmol), **compound 3** (0.11 g, 0.60 mmol), and DMAP (4.3 mg, 0.35 mmol) were added. The solution was stirred overnight at room temperature, and then filtrated to remove DCU. The solution was evaporated to remove solvent and purified by column chromatography (Hexane:EA, 7:3) to obtain 270 mg of the product (**compound 13**, yield 70%). $^1$H NMR (600 MHz, $CDCl_3$) δ 5.46 - 5.21 (m, 8H), 4.99 (s,1H), 4.35 (dd, *J* = 12.1, 4.1 Hz, 2H), 4.17 (dd, *J* = 12.1, 5.9 Hz, 2H), 3.95 (d, *J* = 5.2 Hz, 2H), 2.79 (t, *J* = 6.8 Hz, 4H), 2.33 (t, *J* = 7.6 Hz, 4H), 2.07 (q, *J* = 7.0 Hz, 7H), 1.62 (dd, *J* = 15.9, 8.6 Hz, 5H), 1.47 (s, 9H), 1.43 - 1.17 (m, 28H), 0.91 (t, *J* = 6.9 Hz, 6H).

***Preparation of* (9Z,9'Z,12Z,12'Z)-2-(2-aminoacetoxy)propane-1,3-diyl bis(octadeca-9,12-dienoate) (14)** To a solution of **compound** 13 (0.27 g, 0.35 mmol) dissolved in 5 mL of DCM, TFA (0.53 mL, 6.98 mmol) was added slowly and stirred until reactant was consumed. The solution was evaporated to remove solvent. The residue was washed with ether and hexane, and the residual solvent was removed by evaporation under reduced pressure, thereby obtaining about 200 mg of orange gluey product (**compound 14**, yield 85%). $^1$H NMR (600 MHz, MeOD) δ 5.43 - 5.28 (m, 8H), 4.43 (dd, J = 12.2, 4.2 Hz, 2H), 4.26 (dd, J = 12.2, 5.7 Hz, 2H), 3.88 (s, 2H), 2.79 (t, J = 6.6 Hz, 4H), 2.37 (t, J = 7.4 Hz, 4H), 2.15 - 2.03 (m, 8H), 1.68 - 1.58 (m, 4H), 1.44 - 1.25 (m, 28H), 0.92 (t, J = 7.0 Hz, 6H). LC-MS (ESI); m/z: $[M+H]^+$ Calcd. for $C_{41}H_{71}NO_6$, 673.53. Found 675.91.

### 1.5 MGG-linoleic acid (Compound 18)

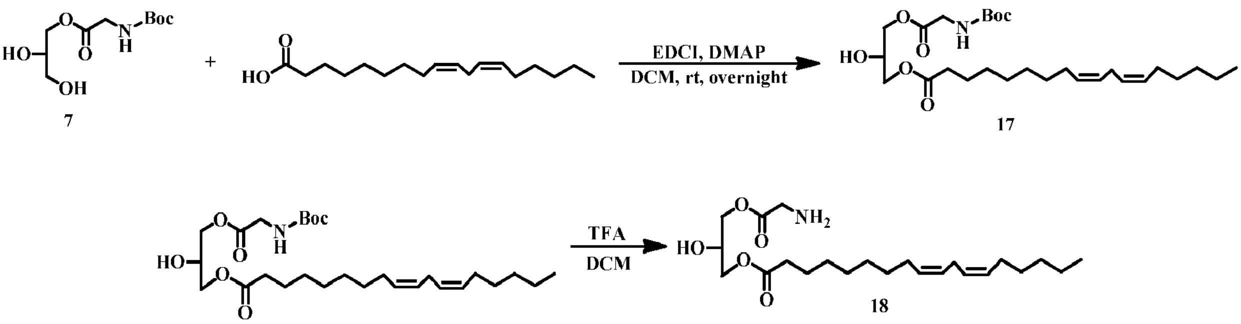


7 17 18

***Preparation of (9Z,12Z)-3-(2-((tert-butoxycarbonyl)anuno)acetoxy)-2-hydroxypropyl octadeca-9,12-dienoate (17)*** **Compound 7** (0.19 g, 0.75 mmol), linoleic acid (0.1 g, 0.36 mmol), 1-Ethyl-3-(3-dimethylaminopropyl)carbodiimide (EDCI, 0.11 g, 0.73 mmol) and DMAP (44 mg, 0.36 mmol) were mixed and dissolved in 10 mL of DCM in a round bottom bottle. The solution was stirred at room temperature overnight, and then evaporated to remove DCM. Crude product was purified by column chromatography (Hex:EA, 2:3) to obtain 75 mg of sticky liquid product (**compound 17**, yield 41%). $^1$H NMR (600 MHz, CDCl3) δ 5.47 - 5.30 (m, 2H), 4.50 - 4.01 (m, 5H), 3.96 (d, J = 5.4 Hz, 2H), 2.79 (t, J = 6.9 Hz, 1H), 2.37 (t, J = 7.5 Hz, 2H), 2.07 (dd, J = 13.4, 7.2 Hz, 2H), 1.63 (dd, J = 23.5, 18.2 Hz, 8H), 1.53 - 1.38 (m, 9H), 1.45 - 1.19 (m, 14H), 0.99 - 0.85 (m, 3H).

***Preparation of (9Z,12Z)-3-(2-aminoacetoxy)-2-hydroxypropyl octadeca-9,12-dienoate (18)*** To a solution of **compound 17** (75 mg, 0.15 mmol) dissolved in 4 mL of DCM, TFA (0.22 mL, 2.93 mmol) was added slowly and stirred until the reactant was consumed, then the solution was evaporated to remove DCM. The residue was washed with ether and DCM. The remained solvent was then evaporated again under reduced pressure, thereby obtaining about 75 mg of light brown gluey product (**compound 18**, yield 100%). $^1$H NMR (600 MHz, MeOD) δ 5.43 - 5.29 (m, 4H), 4.33

(dd, J = 11.4, 4.3 Hz, 1H), 4.28 (dd, J = 11.4, 5.8 Hz, 1H), 4.15 (dd, J = 5.4, 3.8 Hz, 1H), 4.11 - 4.03 (m, 1H), 3.89 (s, 2H), 2.79 (t, J = 6.6 Hz, 2H), 2.38 (t, J = 7.5 Hz, 2H), 2.11 - 2.05 (m, 4H), 1.68 - 1.59 (m, 2H), 1.41 - 1.28 (m, 14H), 0.92 (t, J = 7.0 Hz, 3H). LC-MS (ESI); m/z: [M+H]+ Calcd. for $C_{23}H_{41}NO_5$,411.30. Found 412.98.

**1.6 TGG-di(palmitoleic acid) (Compound 22)**

*Procedure 1*

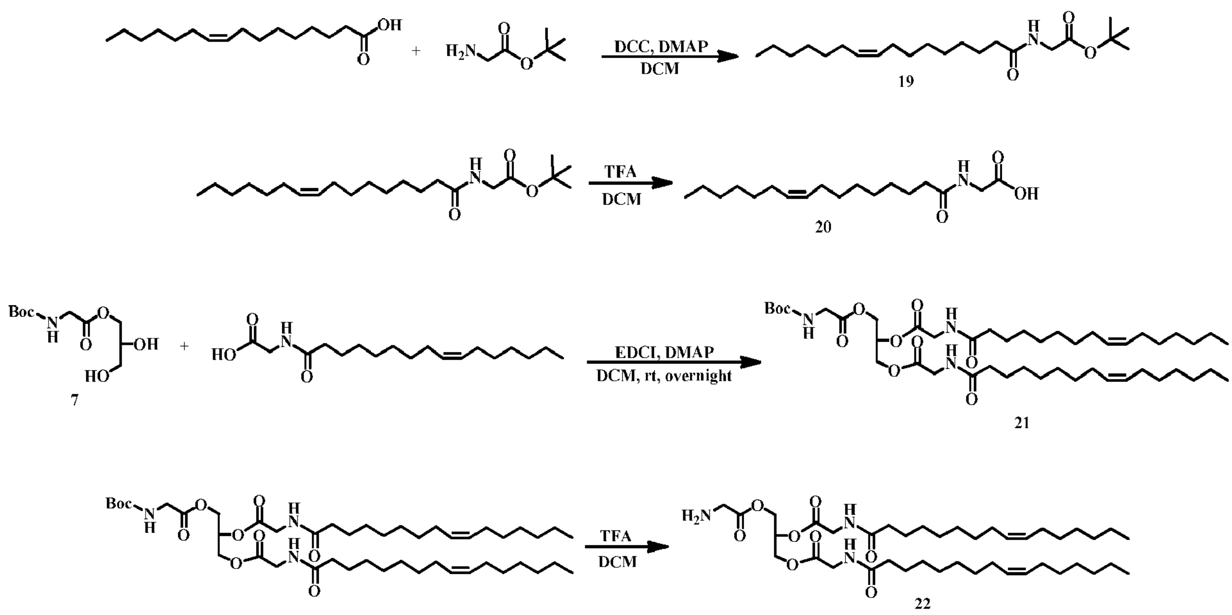

19
20
7
21
22

***Preparation of (Z)-2-(2-(hexadee-9-enamido)acetoxy)propane-1,3-diyl bis(2-((tert-butoxycarbonyl) amino) acetate) (19)***

To a solution of DCC (0.97 g, 4.72 mmol) dissolved in 12 mL of DCM in a round bottom bottle, palmitoleic acid (1 g, 3.93 mmol), t-butyl 2-aminoacetate (0.62 g, 4.72 mmol), and DMAP (0.34 g, 2.75 mmol) were added. The solution was stirred at room temperature overnight, then was filtered to remove DCU and evaporated to remove solvent. Crude product was purified by column chromatography (Hexane:EA, 3:1) to obtain about 1.04 g of clear sticky liquid product **(compound 19,** yield 72%). $^1$H NMR (600 MHz, $CDCl_3$) δ 5.92 (s, 1H), 5.36 (ddd, *J* = 7.5, 3.9, 1.9 Hz, 2H), 3.96 (d, *J* = 4.9 Hz, 2H), 2.24 (dd, *J* = 10.1, 5.3 Hz, 2H), 2.03 (q, *J* = 6.5 Hz, 3H), 1.66 (dd, *J* = 14.5, 7.7 Hz, 2H), 1.49 (s, 9H), 1.31 (ddd, *J* = 21.0, 13.6, 7.1 Hz, 16H), 0.90 (dd, *J* = 9.7, 4.4 Hz, 3H).

***Preparation of (Z)-2-(hexadec-9-enamido)acetic acid (20)*** To a solution of **compound 19** (1.04 g, 2.83 mmol) dissolved in DCM, TFA (4.33 mL, 56.59 mmol) was added slowly and stirred until the reactant was consumed. The solution was evaporated to remove DCM. The residue was washed with ether and hexane, and the remained solvent was removed again by evaporation under reduced pressure, so as to provide a white powder product (about 870 mg, **compound 20**) for further use (Yield 99%). $^1$H NMR (600 MHz, MeOD) δ 5.36 (t, J = 4.7 Hz, 2H), 3.90 (s, 2H), 2.26 (t, J = 7.5 Hz, 2H), 2.04 (dd, J = 11.9, 6.2 Hz, 4H), 1.73 - 1.60 (m, 2H), 1.33 (dd, J = 14.6, 8.6 Hz, 16H), 0.92 (t, J = 7.0 Hz, 3H).

***Preparation of (Z)-3-(2-((tert-butoxycarbonyl)amino)acetoxy)propane-1,2-diyl bis(2-((Z)-hexadec-9-enamido)acetate) (21)*** **Compound 7** (50 mg, 0.20 mmol), **compound 20** (0.14 g, 0.44 mmol), EDCI (70 mg, 0.44 mmol), and DMAP (25 mg, 0.20 mmol) were mixed and added in 10 ml of DCM in a round bottom bottle. The solution was stirred overnight at room temperature overnight., then was evaporated under reduced pressure to remove DCM. Crude product was purified by column chromatography (Hex:EA, 1:4) to obtain 140 mg of orange sticky liquid product (**compound 21**, yield 84%). $^1$H NMR (600 MHz, $CDCl_3$) δ 5.43 - 5.33 (m, 2H), 4.41 - 4.32 (m, 2H), 4.11 - 4.03 (m, 4H), 3.95 - 3.86 (m, 2H), 2.28 (tt, *J* = 13.8, 6.9 Hz, 4H), 2.07 - 1.99 (m, 4H), 1.92 (tdd, *J* = 20.5, 12.6, 7.8 Hz, 2H), 1.86 - 1.76 (m, 2H), 1.75 - 1.51 (m, 12H), 1.48 (s, 9H), 1.44 - 1.21 (m, 32H), 0.91 (q, *J* = 6.9 Hz, 6H).

***Preparation of (Z)-3-(2-aminoacetoxy)propane-1,2-diyl bis(2-((Z)-hexadec-9-enamido)acetate) (22)*** To a solution of compound 21 (0.14 g, 0.17 mmol) dissolved in 4 mL of DCM, TFA (0.26 mL, 3.35 mmol) was added slowly and stirred until reactant was consumed completely. The solution was then evaporated to remove DCM, and the residue

was washed with hexane and DCM. The remained solvent was further removed by evaporation under reduced pressure. Thus, about 80 mg of orange gluey product was obtained (**compound 22**, yield 64%). $^1$H NMR (600 MHz, MeOD) δ 5.37 (dd, *J* = 19.9, 15.0 Hz, 5H), 4.61 - 4.24 (m, 4H), 4.04 - 3.79 (m, 6H), 2.27 (td, *J* = 7.5, 4.0 Hz, 4H), 2.17 - 1.92 (m, 8H), 1.70 - 1.55 (m, 4H), 1.49 - 1.23 (m, 32H), 0.92 (t, *J* = 7.0 Hz, 6H). LC-MS (ESI); m/z: $[M+H]^+$ Calcd. for $C_{41}H_{73}N_3O_8$, 735.54. Found 737.33.

*Procedure 2*

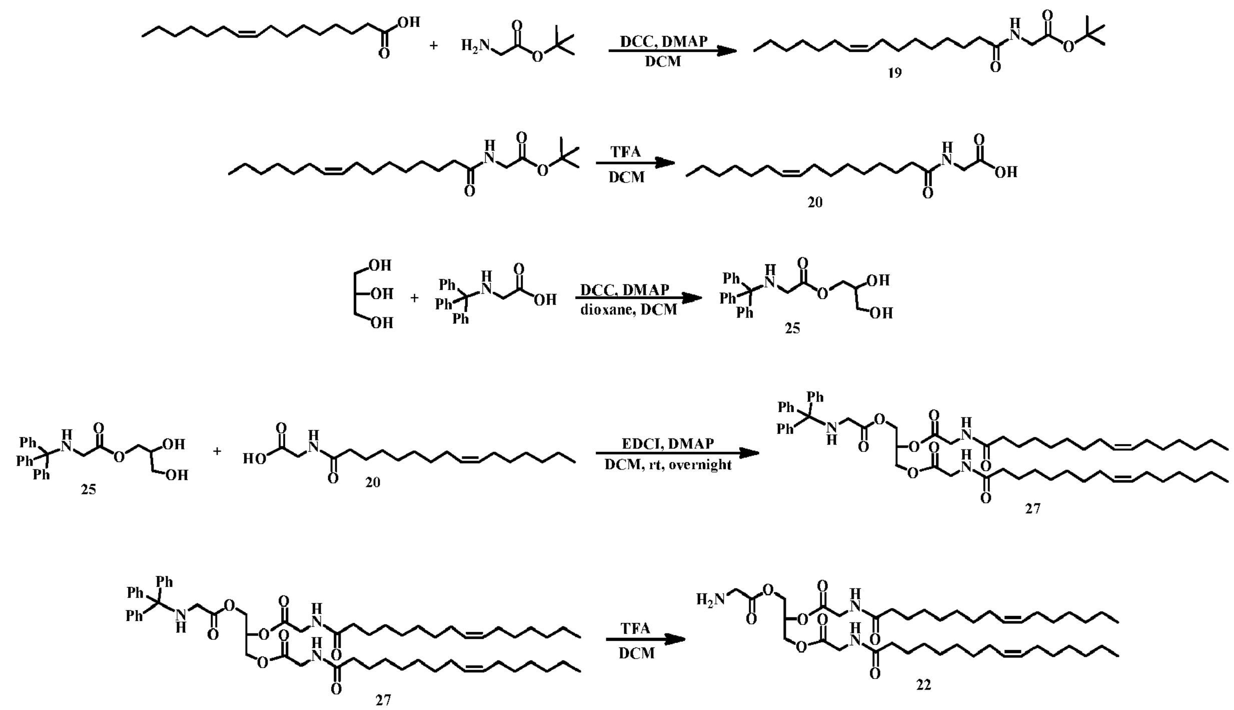


***Preparation of (Z)-2-(2-(hexadee-9-enamido)acetoxy)propane-1,3-diyl bis(2-((tert-butoxycarbonyl) amino) acetate) (19)*** To a solution of DCC (1.16 g, 5.66 mmol) dissolved in 10 ml of DCM in a round bottom bottle, palmitoleic acid (1.2 g, 4.72 mmol), t-butyl 2-aminoacetate (0.74 g, 5.66 mmol), and DMAP (0.4 g, 3.3 mmol) were added gradually and stirred at room temperature overnight. The mixture was filtered to remove DCU and evaporated to remove solvent. Crude product was purified by column chromatography (Hexane:EA, 4:1) to obtain about 1.37 g of clear sticky liquid product **(compound 19,** yield 72%).

***Preparation of (Z)-2-(hexadec-9-enamido)acetic acid (20)*** To a solution of **compound 19** (1.37 g, 3.73 mmol) dissolved in DCM, TFA (4.28 mL, 55.91 mmol) was added slowly and stirred until the reactant was consumed. The solution was evaporated to remove DCM. The residue was washed with ether and hexane, and the remained solvent was removed again by evaporation under reduced pressure, so as to provide a white powder product (about 1150 mg, **compound 20**) for further use (Yield 99%).

***Preparation of 3-(2-(tritylamino)acetoxy)propane-1,2-diyl bis(2-((9Z,12Z)-octadeca-9,12-dienamido)acetate) (25)*** Glycerol (0.29 g, 3.15 mmol), trt-glycine (0.2 g, 0.63 mmol), were placed in a round bottom bottle and the mixture of DCM/dioxane was added as solvent. DCC (0.17 g, 0.82 mmol) and DMAP (38 mg, 0.32 mmol) were added into the round bottom bottle gradually. DCU was removed after the solution was stirred overnight at room temperature. Solvent was removed under reduced pressure. Crude product was purified by column chromatography (Hex:EA = 1:3) to obtain 195 mg white solid product (yield 79%).

***Preparation of (Z)-3-(2-(tritylamino)acetoxy)propane-1,2-diyl bis(2-((Z)-hexadec-9-enamido)acetate) (27)*** **Compound 25** (0.2 g, 0.51 mmol), **compound 20** (0.37 g, 1.18 mmol), EDCI (0.18 g, 1.18 mmol), and DMAP (60 mg, 0.51 mmol) were mixed and dissolved in 10 ml of DCM, and the mixture was stirred at room temperature overnight. The mixture was then evaporated to remove DCM to obtain crude product. The crude product then was purified by column chromatography (Hex:EA, 3:2) to obtain 280 mg of white viscous product (**compound 27**, yield 56%). $^1$H NMR (600 MHz, $CDCl_3$) δ 7.49 (d, J = 7.6 Hz, 6H), 7.36 (s, 5H), 5.37 (dd, J = 9.6, 4.3 Hz, 2H), 5.32 - 5.20 (m, 1H), 4.31 - 4.20 (m, 3H), 4.15 (s, 1H), 4.09 - 4.02 (m, 4H), 3.96 (dd, J = 18.1, 5.3 Hz, 1H), 2.27 (td, J = 7.7, 3.9 Hz, 4H), 2.08 - 1.97 (m,

4H), 1.97 - 1.87 (m, 2H), 1.86 - 1.75 (m, 2H), 1.63 (dd, J = 23.3, 15.7 Hz, 14H), 1.49 - 1.20 (m, 30H), 0.91 (q, J = 6.9 Hz, 6H).

***Preparation of (Z)-3-(2-aminoacetoxy)propane-1,2-diyl bis(2-((Z)-hexadec-9-enamido)acetate) (22)*** To a solution of **compound 27** (0.28 g, 0.29 mmol) dissolved in 8 mL of DCM, TFA (0.33 mL, 4.29 mmol) was added slowly and stirred until reactant was consumed completely. The solution was evaporated to remove solvent, and the residue was purified by column chromatrography (DCM:MeOH, 95:5 to 90:10) to obtain about 200 mg of white to pale yellow viscous solid product (**compound 22**, yield 94%). $^1$H NMR (600 MHz, MeOD) δ 5.40 (tt, *J* = 6.3, 4.1 Hz, 1H), 5.37 - 5.29 (m, 3H), 4.51 - 4.43 (m, 2H), 4.37 (tt, *J* = 18.1, 6.2 Hz, 2H), 4.05 - 3.76 (m, 6H), 2.26 (td, *J* = 7.5, 4.1 Hz, 4H), 2.02 (dd, *J* = 20.5, 13.2 Hz, 7H), 1.71 - 1.55 (m, 5H), 1.46 - 1.17 (m, 32H), 0.90 (t, *J* = 7.0 Hz, 6H). LC-MS (ESI); m/z: $[M+H]^+$ Calcd. for $C_{41}H_{73}N_3O_8$, 735.54. Found 737.18.

### 1.7 TGG-palmitoleic acid (Compound 24)

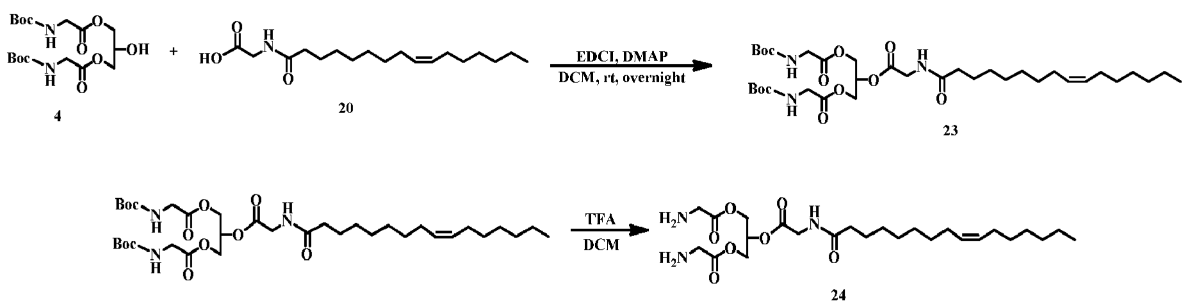


***Preparation of (Z)-2-(2-(hexadee-9-enamido)acetoxy)propane-1,3-diyl bis(2-((tert-butoxycarbonyl) amino) acetate) (23)*** **Compound 4** (0.22 g, 0.54 mmol), **compound 20** (0.20 g, 0.65 mmol), EDCI (0.10 g, 0.65 mmol), and DMAP (0.07 g, 0.54 mmol) were mixed and dissolved in 10 ml of DCM in a round bottom bottle. The mixture was stirred at room temperature overnight, and then evaporated to remove DCM. The residue was extracted with EA/$H_2O$, and organic layer was collected, dried over $MgSO_4$, and filtered. The filtrate was evaporated under reduced pressure to remove the solvent. Crude product was purified by column chromatography (Hexane:EA, 2:3) to obtain 290 mg of clear sticky liquid product **(compound 23,** yield 77%). $^1$H NMR (600 MHz, CDCl3) δ 5.43 - 5.31 (m, 2H), 5.21 (s, 1H), 4.50 - 4.35 (m, 2H), 4.30 (dd, J = 15.4, 8.4 Hz, 2H), 4.14 - 3.96 (m, 3H), 3.98 - 3.81 (m, 3H), 2.27 (ddd, J = 15.2, 7.3, 3.2 Hz, 2H), 2.03 (dd, J = 13.1, 6.7 Hz, 2H), 1.92 (ddd, J = 19.1, 9.5, 4.8 Hz, 1H), 1.87 - 1.75 (m, 1H), 1.75 - 1.51 (m, 8H), 1.47 (s, 15H), 1.44 - 1.23 (m, 16H), 0.91 (q, J = 6.9 Hz, 3H).

***Preparation of (Z)-2-(2-(hexadee-9-enamido)acetoxy)propane-1,3-diyl bis(2-aminoacetate) (24)*** To a solution of **compound 23** (0.29 g, 0.41 mmol) dissolved in 5 mL of DCM, TFA (0.95 mL, 12.43 mmol) was added slowly and stirred until all the reactant was consumed. After DCM in solution was removed by rotary evaporator, the residue was washed by hexane and ether. The remained solvent was removed by evaporation under reduced pressure, thereby obtain about 170 mg of clear sticky product (**compound 24**, yield 83%). $^1$H NMR (600 MHz, MeOD) δ 5.51 - 5.45 (m, 1H), 5.36 (dd, *J* = 7.5, 3.4 Hz, 2H), 4.57 - 4.44 (m, 3H), 3.94 - 3.88 (m, 5H), 2.33 - 2.22 (m, 2H), 2.05 (dt, *J* = 12.1, 4.2 Hz, 4H), 1.63 (dd, *J* = 14.4, 7.2 Hz, 2H), 1.45 - 1.22 (m, 16H), 0.91 (t, *J* = 7.0 Hz, 3H). LC-MS (ESI); m/z: $[M+H]^+$ Calcd. for $C_{25}H_{45}N_3O_7$ 499.33. Found 501.12.

## Example 2 The present cationic lipids encapsulate and help delivering nucleic acids

### 2.1 Encapsulation of nucleic acids

In this example, the ability for the present cationic lipids for protecting nucleic acids was investigated. To this purpose, the cationic lipids of Example 1 (*i.e.*, compounds 6, 9, 11, 14, 18, 22, or 24) were independently co-cultivated with plasmids, then subjected to DNA digestion and extraction by following the procedures described in "Materials and Methods" section. Samples were analyzed via agarose gel electrophoresis, and results are provided in FIG. 1.

It was evident from the gel electrophoresis that, after digestion, all detectable nucleotides were extracted and derived from the liposomes (i.e., the cationic lipid-DNA complexes), indicating that the present cationic lipids are able to encapsulate and protect plasmids from the action of nucleases (lanes 25-28, and 31-34 of FIG.1).

Further, the encapsulation ratio of the present cationic lipids for encapsulating small RNAs was investigated. To this purpose, the commercial cationic lipids and the present cationic lipids (*i.e.*, compounds 6 (TGG-linoleic acid) and 22 (TGG-di(palmitoleic acid)) were respectively mixed with fluorochrome-conjugated small RNAs (i.e., Cy5-mir302a-siRNA, SEQ ID NO: 6 and 7) by following the preparation procedures described in "Materials and Methods" section, thereby producing lipid nanoparticles (LNPs) containing small RNAs therein. The size, zeta potentials, and encapsulation efficiency (EE) of each LNPs were analyzed in accordance with procedures described in "Materials and Methods" section. Results are summarized in Table 1.

Table 1 Characterization of the present LNPs

| Sample | Size (nm) | Zeta potential (mV) | EE (%) |
|---|---|---|---|
| Control LNPs (D-lin-MC3-DMA (MC30)) | 123.6 | -4.30 | 96.08 |
| Present LNPs (compound 6, TGG-linoleic acid) | 125.3 | -2.10 | 89.33 |
| Present LNPs (compound 22, TGG-di(palmitoleic acid) | 68.35 | -5.76 | 98.31 |

The data in Table 1 revealed that both compounds 6 and 22 successfully formed liposomes and encapsulated siRNAs therein. Specifically, as compared with the LNPs made by commercial cationic lipids MC30, the present LNPs composed of the present compound 22 had a smaller size and a higher encapsulation efficiency.

**2. 2 Delivery of nucleic acids**

In this example, whether the present cationic lipids may promote the delivery of nucleic acids into cells was investigated. To this purpose, A549 and NCI-H460 lung cancer cell lines were respectively co-cultivated with the present or control LNPs with various concentration of siRNAs (0.25, 1.25 or 2.5 ng/μL) therein for 24 hours, and the transfection efficiency was verified by flow cytometry. Results are depicted in FIGs. 2A to 2B.

It is evident from the flow cytometry histograms in FIGs. 2A and 2B that, after co-cultivating with the present LNPs composed of compounds 6 or 22, the florescence intensity emitted from lung cancer cells gradually increased, indicating that external nucleic acids had been successfully transfected into cancer cells. Further, the peaks shifted to the rightmost histograms of LNPs composed of the present compound 22 (TGG-di(palmitoleic acid)) indicating that it possessed better delivery efficacy than that of the LNPs composed of compound 6.

The data set forth above collectively indicates that the present novel cationic lipids are capable of encapsulating functional nucleic acids therein, thus, the lipid nanoparticles assembled by the present cationic lipids can serve as a carrier for the delivery of therapeutic agents into cells, thereby achieving effective gene editing, cell engineering, and disease treatment.

It will be understood that the above description of embodiments is given by way of example only and that various modifications may be made by those with ordinary skill in the art. The above specification, examples, and data provide a complete description of the structure and use of exemplary embodiments of the invention. Although various embodiments of the invention have been described above with a certain degree of particularity, or with reference to one or more individual embodiments, those with ordinary skill in the art could make numerous alterations to the disclosed embodiments without departing from the spirit or scope of this invention.

## Claims

1. A cationic lipid of formula (I),

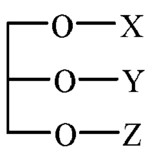

(I),

wherein

at least one of X and Y is $-(C{=}O)CH_2NH_2$, and the other X, Y, and Z are independently selected from the group consisting of $-(C{=}O)CH_2NH_2$, $-[(C{=}O)CH_2NH](C{=}O)R^1$, $-(C{=}O)R^1$, and H, wherein $R^1$ is $C_{9-25}$ alkyl, or $C_{13-21}$ alkenyl.

2. The cationic lipid of claim 1, wherein X and Z are independently $-(C{=}O)CH_2NH_2$, Y is - $[(C{=}O)CH_2NH](C{=}O)R^1$, and $R^1$ is $C_{13-21}$ alkenyl.

**3.** The cationic lipid of claim 1, wherein X is $-(C{=}O)CH_2NH_2$, Y and Z are independently - $[(C{=}O)CH_2NH](C{=}O)R^1$, and $R^1$ is $C_{13\text{-}21}$ alkenyl.

**4.** The cationic lipid of claim 1, wherein Y is $-(C{=}O)CH_2NH_2$, X and Z are independently - $(C{=}O)R^1$, and $R^1$ is $C_{13\text{-}21}$ alkenyl.

**5.** The cationic lipid of claim 1, wherein X and Z are independently $-(C{=}O)CH_2NH_2$, Y is - $(C{=}O)R^1$, and $R^1$ is $C_{13\text{-}21}$ alkenyl.

**6.** The cationic lipid of claim 1, wherein X is $-(C{=}O)CH_2NH_2$, Y is H, Z is $-(C{=}O)R^1$, and $R^1$ is $C_{13\text{-}21}$ alkenyl.

**7.** The cationic lipid of claim 1, wherein the cationic lipid of formula (I) is selected from the group consisting of

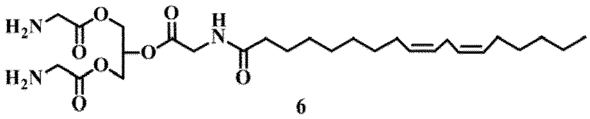

6

,

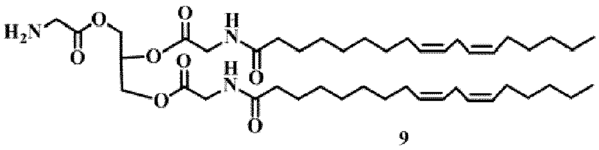

9

,

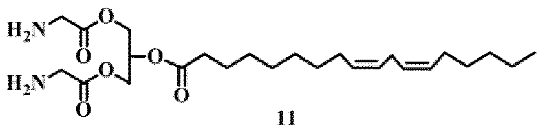

11

,

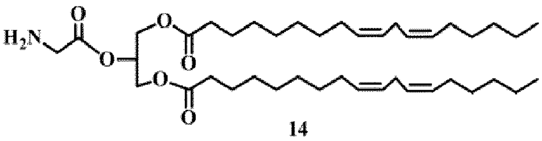

14

,

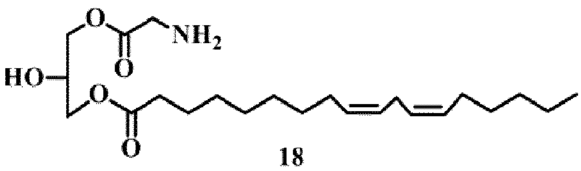

18

,

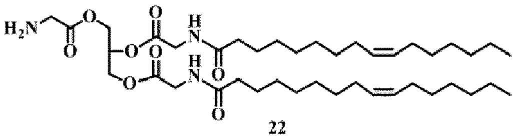

22

, and

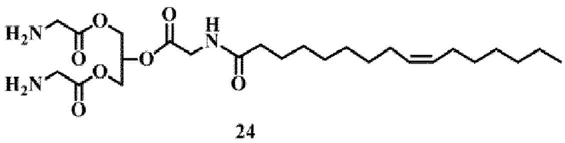

24

.

**8.** A lipid nanoparticle (LNP) comprising a cationic lipid of claim 1, a non-cationic lipid, and an active pharmaceutical ingredient (API).

**9.** The LNP of claim 8, wherein the API is a nucleic acid, a peptide, a polypeptide, a protein, a carbohydrate, a proteoglycan, a glycoprotein, or a combination thereof.

**10.** The LNP of claim 9, wherein the nucleic acid is a mitochondrial DNA (mtDNA), a chloroplast DNA (cpDNA), a plasmid, a messenger RNA (mRNA), a small interfering RNA (siRNA), a ribosomal RNA (rRNA), a transfer RNA (tRNA), a microRNA (miRNA), or an aptamer.

**11.** The LNP of claim 10, wherein the miRNA is a precursor miRNA or a mature miRNA.

**12.** The LPN of claim 8, wherein the cationic lipid is selected from the group consisting of

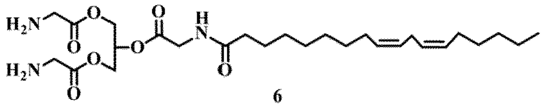

6

,

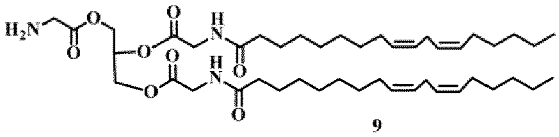

9

,

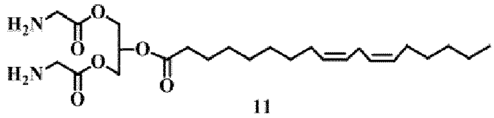

11

,

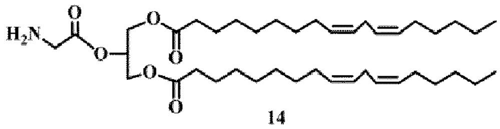

14

,

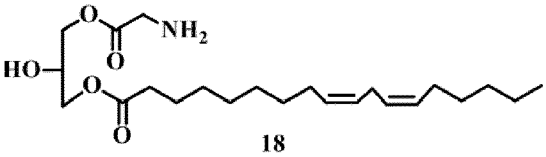

18

,

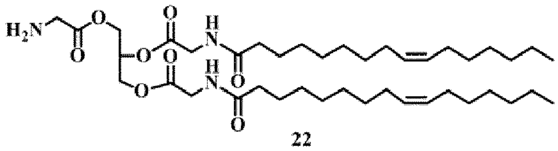

22

,

and

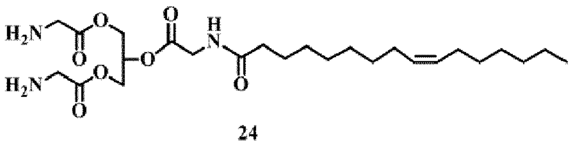

24

;

and the API is the miRNA comprising a sequence of SEQ ID NO:1.

**13.** The LNP of claim 12, wherein the miRNA has a sequence of SEQ ID NO:2, 3, 4, or 5.

**14.** The LNP of claim 8, wherein the cationic lipid is

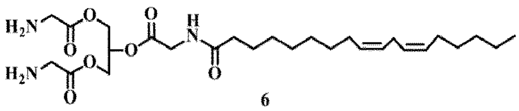

6

or

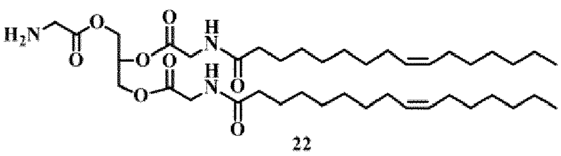

22

;

and the API is the siRNA having a sequence of SEQ ID NO: 6 or 7.

**15.** The LNP of claim 8, wherein each of the non-cationic lipids is selected from the group consisting of a helper lipid, a phospholipid, a PEGylated lipid, a PEGylated phospholipid, and a combination thereof.

**16.** The LNP of claim 15, wherein the helper lipid is dioleoylphosphatidylethanolamine (DOPE), dipalmitoylphosphatidylcholine (DPPC), distearoylphosphatidylethanolamine (DSPE), dimyristoylphosphatidylethanolamine (DMPE), or distearoylphosphatidylcholine (DSPC).

**17.** The LNP of claim 16, wherein the helper lipid is DSPC.

**18.** The LNP of claim 8, wherein the cationic lipid and the non-cationic lipid are respectively present in the LNP at a molar ratio of 1:5 to 5:1.

**19.** The LNP of claim 8, wherein the LNP further comprises a steroid, which is selected from the group consisting of cholesterol, lanosterol, ergosterol, phytosterol, stigmasterol, and brassicasterol.

**20.** The LNP of claim 19, wherein the steroid is cholesterol.

**21.** The LNP of claim 8 for use as a medicament for treating a disease in a subject in need thereof.

**22.** The use of claim 21, wherein the disease is a cancer, an infectious disease, or an autoimmune disease.

**23.** The use of claim 22, wherein the cancer is selected from the group consisting of a bladder cancer, a bone cancer, a bone marrow cancer, a brain cancer, a breast cancer, a cholangiocarcinoma, a colon cancer, an esophagus cancer, a gastrointestinal cancer, a gum cancer, a head and neck cancer, a kidney cancer, a liver cancer, a lung cancer, a nasopharyngeal carcinoma, a leukemia, a lymphoma, an ovary cancer, a prostate cancer, a skin cancer, a stomach cancer, a testis cancer, a tongue cancer, and a uterus cancer.

**24.** The use of claim 23, wherein the cancer is the lung cancer, the cationic lipid is selected from the group consisting of

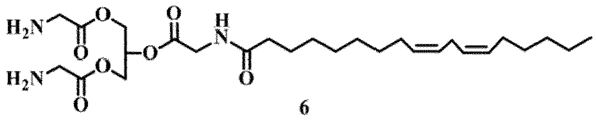

6

,

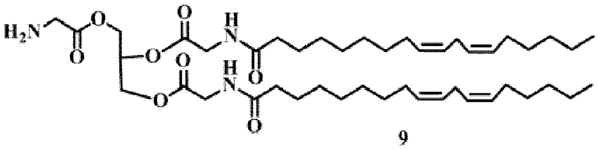

9

,

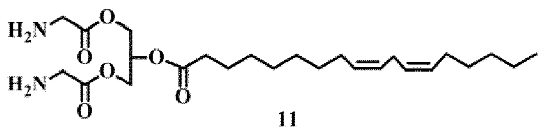

11

,

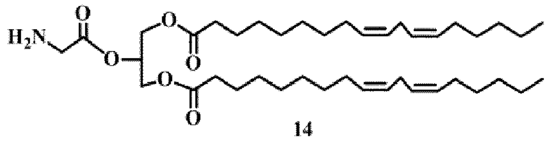

14

,

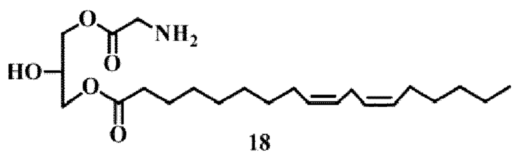

18

,

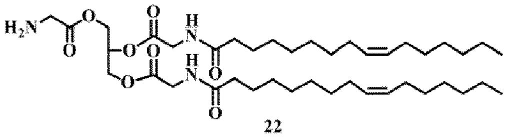

22

, and

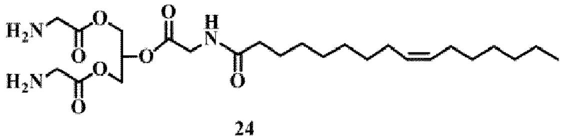

24

;

and the API is a miRNA comprising a sequence of SEQ ID NO:1.

**25.** The use of claim 24, wherein the miRNA has a sequence of SEQ ID NO:2, 3, 4, or 5.

**26.** The use of claim 23, wherein the cancer is the lung cancer, the cationic lipid is

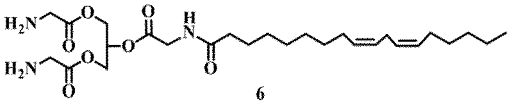

6

or

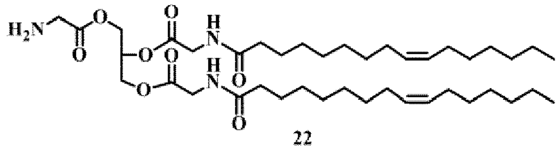

22

;

and the API is a siRNA having a sequence of SEQ ID NO: 6 or 7.

**27.** The use of claim 21, wherein the subject is a human.

**28.** A method for delivering an API into a cultivated cell, comprising contacting the cultivated cell with an effective amount of the LNP of claim 8, wherein the API is a nucleic acid, a polypeptide, a protein, a carbohydrate, a proteoglycan, a glycoprotein, or a combination thereof.

**29.** The method of claim 28, wherein the nucleic acid is a mitochondrial DNA (mtDNA), a chloroplast DNA (cpDNA), a plasmid, a messenger RNA (mRNA), a small interfering RNA (siRNA), a ribosomal RNA (rRNA), a transfer RNA (tRNA), a microRNA (miRNA), or an aptamer.

**30.** The method of claim 29, wherein the miRNA is a precursor miRNA or a mature miRNA.

**31.** The method of claim 28, wherein the cultivated cell is a stem cell, an immune cell, or a combination thereof.

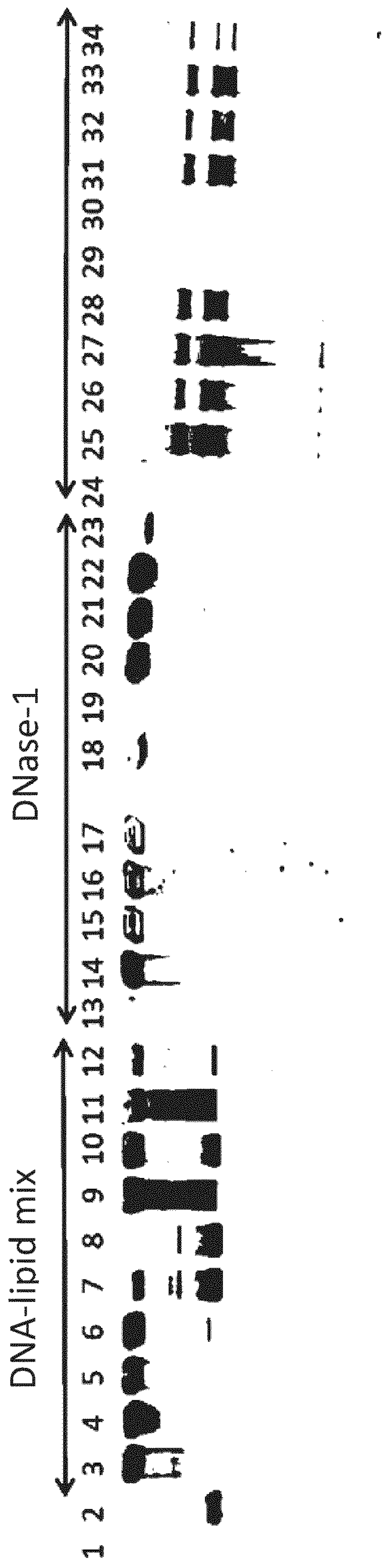
DNA-lipid mix
DNase-1
1 2 3 4 5 6 7 8 9 10 11 12 13 14 15 16 17 18 19 20 21 22 23 24 25 26 27 28 29 30 31 32 33 34

FIG. 1

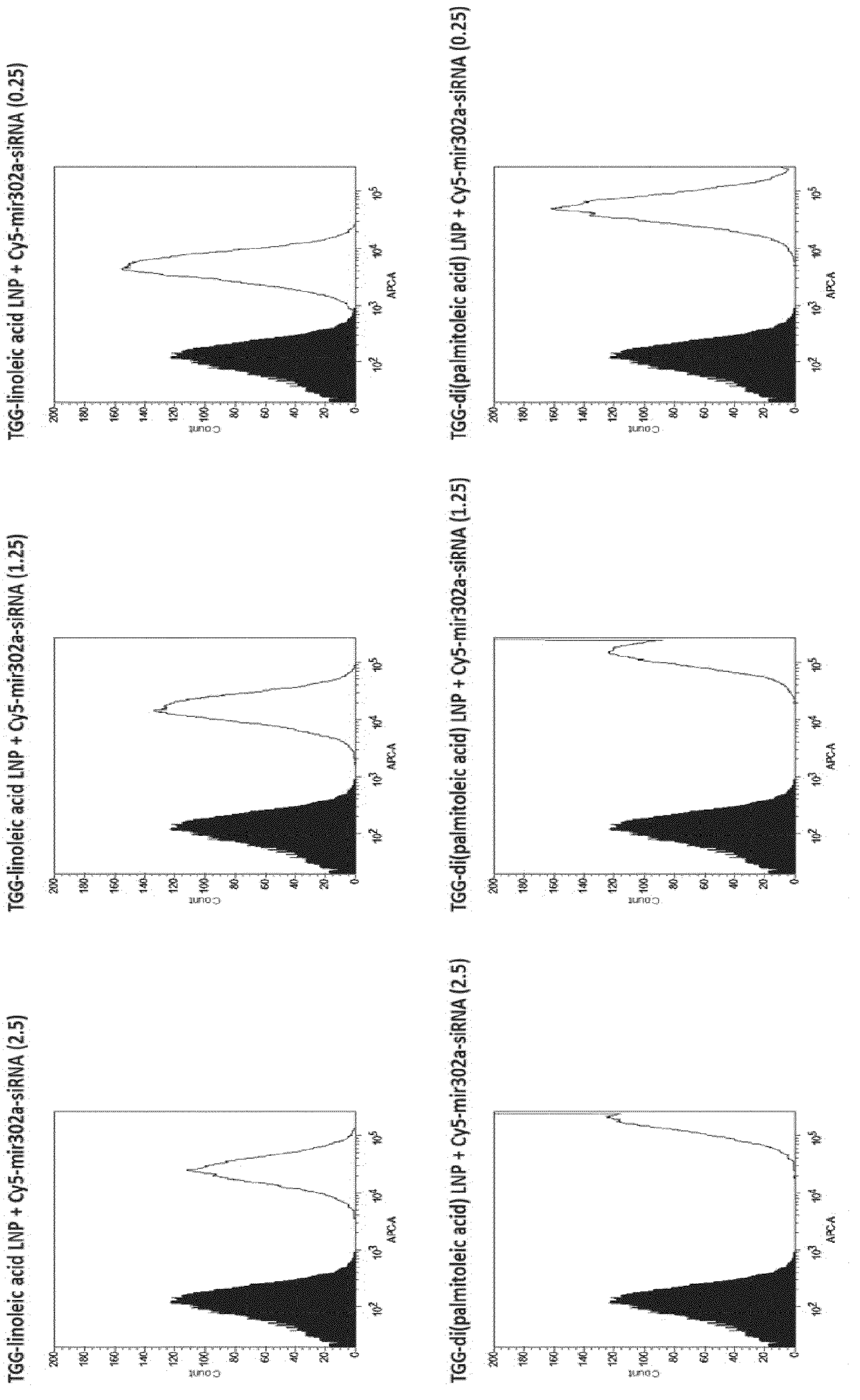
TGG-linoleic acid LNP + Cy5-mir302a-siRNA (0.25)
TGG-linoleic acid LNP + Cy5-mir302a-siRNA (1.25)
TGG-linoleic acid LNP + Cy5-mir302a-siRNA (2.5)
TGG-di(palmitoleic acid) LNP + Cy5-mir302a-siRNA (0.25)
TGG-di(palmitoleic acid) LNP + Cy5-mir302a-siRNA (1.25)
TGG-di(palmitoleic acid) LNP + Cy5-mir302a-siRNA (2.5)
Count
APC-A

FIG. 2A

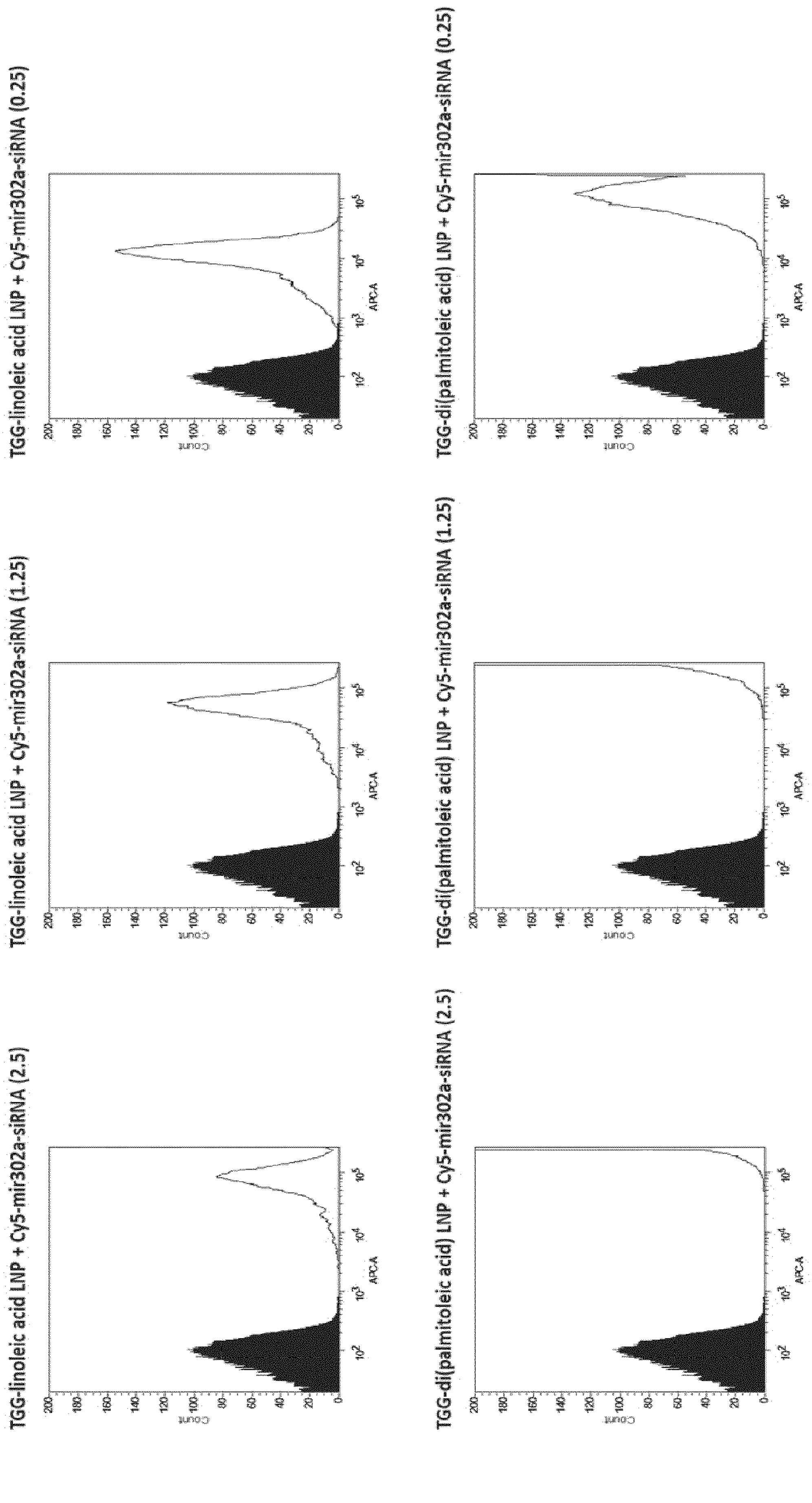
TGG-linoleic acid LNP + Cy5-mir302a-siRNA (0.25)
TGG-linoleic acid LNP + Cy5-mir302a-siRNA (1.25)
TGG-linoleic acid LNP + Cy5-mir302a-siRNA (2.5)
TGG-di(palmitoleic acid) LNP + Cy5-mir302a-siRNA (0.25)
TGG-di(palmitoleic acid) LNP + Cy5-mir302a-siRNA (1.25)
TGG-di(palmitoleic acid) LNP + Cy5-mir302a-siRNA (2.5)
Count
APC-A

FIG. 2B

Europäisches Patentamt
European Patent Office
Office européen des brevets

# EUROPEAN SEARCH REPORT

Application Number
EP 23 20 7899

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 91/16880 A1 (MAX PLANCK GESELLSCHAFT [DE]) 14 November 1991 (1991-11-14)<br>* page 8, lines 15-23 *<br>* page 9, lines 4-10 *<br>* page 18, lines 13-23 *<br>* page 19; compound 9 *<br>* page 24, line 5 - page 25, line 10 *<br>* page 9, lines 25-31 *<br>* page 3, lines 3-16; claims 1-11 *<br>----- | 1-31 | INV.<br>C07C229/08<br>C07C233/49<br>A61K9/51<br>A61P35/00 |
| X | LAMBERT DIDIER M ET AL: "Anticonvulsant activity of ester- and amide-type lipid conjugates of glycine and N-benzyloxycarbonylglycine", EUROPEAN JOURNAL OF PHARMACEUTICAL SCIENCES, ELSEVIER AMSTERDAM, NL, vol. 4, no. 3, 1996, pages 159-166, XP002680155, ISSN: 0928-0987, DOI: 10.1016/0928-0987(95)00044-5<br>* page 163, figure 2, compounds 5 and 7 *<br>----- | 1,4 | |
| | | | TECHNICAL FIELDS SEARCHED (IPC) |
| X | HUBER W F: "MONO-ALPHA-AMINOACYL AND MONO-ALPHA-DIPEPTIDE TRIGLYCERIDES", JOURNAL OF THE AMERICAN CHEMICAL SOCIETY, AMERICAN CHEMICAL SOCIETY, vol. 77, 1955, pages 112-116, XP002008829, ISSN: 0002-7863, DOI: 10.1021/JA01606A036<br>* table IV, first entry *<br>----- | 1,4 | C07C<br>A61K<br>A61P |
| X | WEIZMANN ET AL: "Preparation of glycerides of amino fatty acids", COMPTES RENDUES,, vol. 189, 1929, pages 104-106, XP009552830,<br>* page 105, lines 30-31 *<br>-----<br>-/-- | 1,4 | |

The present search report has been drawn up for all claims

1

| Place of search | Date of completion of the search | Examiner |
|---|---|---|
| The Hague | 20 March 2024 | Fitz, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 20 7899

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/002197 A1 (SOHN KI YOUNG [KR] ET AL) 7 January 2021 (2021-01-07)<br>* paragraphs [0048] and [0049]: compound H *<br>* paragraph [0052], table 1, example 4 *<br>* paragraph [0055], table 4, experiment 14 *<br>----- | 1 | |
| X | WO 2015/112312 A1 (LIN SHI-LUNG [US]; CHANG-LIN SAMANTHA [US] ET AL.) 30 July 2015 (2015-07-30)<br>* figures 2A, 2B, 2C *<br>* page 4, second paragraph *<br>----- | 1 | |
| X | VALIVETY R ET AL: "CHEMO-ENZYMATIC SYNTHESIS OF AMINO ACID-BASED SURFACTANTS", JOURNAL OF THE AMERICAN OIL CHEMISTS SOCIETY, SPRINGER, DE, vol. 74, no. 7, 1997, pages 879-886, XP001037619, ISSN: 0003-021X, DOI: 10.1007/S11746-997-0232-8<br>* figure 2; compound 2c *<br>----- | 1 | TECHNICAL FIELDS SEARCHED (IPC) |
| X | VODOVOZOVA E L ET AL: "Lipid Derivatives of Sarcolysine, Methotrexate, and Rubomycin", BIOORGANICHESKAYA KHIMIYA, IZDATEL'STVO NAUKA, RU, vol. 22, no. 7, 1996, pages 548-556, XP009111427, ISSN: 0132-3423<br>* page 550; compound X *<br>----- | 1 | |
| X | BE 903 416 A (MONSANTO CO) 10 April 1986 (1986-04-10)<br>* examples 15, 16: intermediates *<br>-----<br>-/-- | 1 | |

The present search report has been drawn up for all claims

| Place of search | Date of completion of the search | Examiner |
|---|---|---|
| The Hague | 20 March 2024 | Fitz, Wolfgang |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 20 7899

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WEIZMANN M ET AL: "ESSAIS EN VUE DE PREPARER LES ETHERS-SELS DU GLYCEROL ET DES ACIDES AMINES AINSI QUE LES ETHERS MIXTES D'ACIDES AMINES ET D'ACIDES GRAS. 1re partie. [Attempts to prepare esters of glycerol and amino acids, as the mixed esters of amino acids and fatty acids. 1st part]", BULLETIN DE LA SOCIÉTÉ CHIMIQUE DE FRANCE / 2, SOCIETY FRANCAISE DE CHIMIE , PARIS, FRANCE, vol. 51, no. 4, 1932, pages 59-73, XP000576871, ISSN: 0037-8968 * page 69, line 5 * * page 69, line 29 * ----- | 1 | |
| | | | TECHNICAL FIELDS SEARCHED (IPC) |

The present search report has been drawn up for all claims

1

| Place of search | Date of completion of the search | Examiner |
|---|---|---|
| The Hague | 20 March 2024 | Fitz, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..............................................................................
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 23 20 7899

This annex lists the patent family members relating to the patent documents cited in the above-mentioned European search report.
The members are as contained in the European Patent Office EDP file on
The European Patent Office is in no way liable for these particulars which are merely given for the purpose of information.

20-03-2024

| Patent document cited in search report | Publication date | Patent family member(s) | Publication date |
|---|---|---|---|
| WO 9116880 A1 | 14-11-1991 | AT E107503 T1 | 15-07-1994 |
| | | AU 643282 B2 | 11-11-1993 |
| | | CA 2081119 A1 | 28-10-1991 |
| | | DE 4013632 A1 | 31-10-1991 |
| | | DK 0526531 T3 | 22-08-1994 |
| | | EP 0526531 A1 | 10-02-1993 |
| | | ES 2056648 T3 | 01-10-1994 |
| | | IE 911427 A1 | 06-11-1991 |
| | | JP H05506661 A | 30-09-1993 |
| | | PT 97500 A | 31-01-1992 |
| | | WO 9116880 A1 | 14-11-1991 |
| US 2021002197 A1 | 07-01-2021 | CA 3094947 A1 | 03-10-2019 |
| | | CN 111902389 A | 06-11-2020 |
| | | EP 3750867 A1 | 16-12-2020 |
| | | JP 7162676 B2 | 28-10-2022 |
| | | JP 2021516689 A | 08-07-2021 |
| | | KR 20190112492 A | 07-10-2019 |
| | | US 2021002197 A1 | 07-01-2021 |
| | | WO 2019190137 A1 | 03-10-2019 |
| WO 2015112312 A1 | 30-07-2015 | CN 106413703 A | 15-02-2017 |
| | | JP 6535686 B2 | 26-06-2019 |
| | | JP 2017508001 A | 23-03-2017 |
| | | WO 2015112312 A1 | 30-07-2015 |
| BE 903416 A | 10-04-1986 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 63423506